(19) Europäisches Patentamt
European Patent Office
Office européen des brevets

(11) **EP 3 131 603 B1**

(12) **EUROPÄISCHE PATENTSCHRIFT**

(45) Veröffentlichungstag und Bekanntmachung des Hinweises auf die Patenterteilung:
**24.02.2021 Patentblatt 2021/08**

(21) Anmeldenummer: **15716540.8**

(22) Anmeldetag: **16.04.2015**

(51) Int Cl.:
**A61M 1/36** (2006.01)

(86) Internationale Anmeldenummer:
**PCT/EP2015/058298**

(87) Internationale Veröffentlichungsnummer:
**WO 2015/158843 (22.10.2015 Gazette 2015/42)**

(54) **VORRICHTUNG ZUM ENTFERNEN VON BLUT AUS EINEM EXTRAKORPORALEN BLUTKREISLAUF UNTER DRUCKKONTROLLE**

DEVICE FOR WITHDRAWING BLOOD FROM AN EXTRACORPOREAL BLOOD CIRCUIT WHILE PRESSURE CONTROL IS PERFORMED

DISPOSITIF D'ÉLIMINATION DE SANG À PARTIR D'UN CIRCUIT SANGUIN EXTRACORPOREL SOUS PRESSION CONTRÔLÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.04.2014 DE 102014105473**

(43) Veröffentlichungstag der Anmeldung:
**22.02.2017 Patentblatt 2017/08**

(73) Patentinhaber: **Fresenius Medical Care Deutschland GmbH**
**61352 Bad Homburg (DE)**

(72) Erfinder: **THYS, Martin**
**97508 Grettstadt (DE)**

(74) Vertreter: **Bobbert & Partner**
**Patentanwälte PartmbB**
**Postfach 1252**
**85422 Erding (DE)**

(56) Entgegenhaltungen:
**EP-A1- 2 535 065       EP-A1- 2 535 067**
**WO-A2-2013/017240    DE-A1-102009 027 195**
**JP-A- 2010 184 029**

EP 3 131 603 B1

**Beschreibung**

Die vorliegende Erfindung betrifft eine Steuervorrichtung gemäß Anspruch 1 zum Durchführen eines Verfahrens zum Entfernen von Blut aus einem extrakorporalen Blutkreislauf und/oder aus einer Funktionseinrichtung, welche jeweils zum Zweck einer Blutbehandlung eines Patienten mit einer Blutbehandlungsvorrichtung verbindbar oder verbunden ist bzw. sind. Zudem betrifft sie eine Blutbehandlungsvorrichtung gemäß Anspruch 12. Des Weiteren betrifft die vorliegende Erfindung ein digitales Speichermedium gemäß Anspruch 13, ein Computerprogramm-Produkt gemäß Anspruch 14 sowie ein Computerprogramm gemäß Anspruch 15.

Aus extrakorporalen Blutkreisläufen wird nach ihrem Einsatz im Blutkreislauf verbliebenes Blut nicht zuletzt aus Hygienegründen regelmäßig aus diesem entfernt.

Aus der JP 2010 184029 A ist eine Vorrichtung zur Blutreinigung bekannt.

In der DE 10 2009 027195 A1 ist eine Vorrichtung zur Förderung von Blut in einem extrakorporalen Kreislauf offenbart.

Ein Verfahren zum Entfernen von Blut aus einem extrakorporalen Blutkreislauf sowie Vorrichtungen sind aus der WO 2013/017240 A2 bekannt.

In der EP 2 535 065 A1 ist ein Dialysator offenbart.

Aus der EP 2 535 067 A1 sind eine Blutreinigungsvorrichtung und ein Priming-Verfahren hierfür bekannt.

Eine Aufgabe der vorliegenden Erfindung ist es, eine weitere Steuervorrichtung zum Durchführen eines Verfahrens zum Entfernen von Blut aus einem extrakorporalen Blutkreislauf oder aus einer Funktionseinrichtung nach Beenden einer Blutbehandlungssitzung anzugeben.

Ferner sollen eine Blutbehandlungsvorrichtung, mit welcher das Verfahren durchführbar ist sowie eine zur Durchführung des Verfahrens vorgesehene Steuereinrichtung, ein geeignetes digitales Speichermedium, ein geeignetes Computerprogramm-Produkt und ein geeignetes Computerprogramm angegeben werden.

Die erfindungsgemäße Aufgabe wird durch eine Steuereinrichtung mit den Merkmalen des Anspruchs 1 sowie eine Blutbehandlungsvorrichtung mit den Merkmalen des Anspruchs 12 gelöst. Die erfindungsgemäße Aufgabe wird ferner gelöst durch ein digitales Speichermedium, ein Computerprogramm-Produkt sowie ein Computerprogramm gemäß den Ansprüchen 13, 14 und 15.

Alle mit dem offenbarten Verfahren erzielbaren Vorteile lassen sich in bestimmten erfindungsgemäßen Ausführungsformen ungeschmälert auch mit den erfindungsgemäßen Vorrichtungen erzielen.

Das offenbarte Verfahren ist geeignet und vorgesehen zum Entfernen von Blut aus einem extrakorporalen Blutkreislauf, welcher wenigstens einen arteriellen Leitungsabschnitt und wenigstens einen venösen Leitungsabschnitt aufweist, und/oder zum Entfernen von Blut aus einer Funktionseinrichtung, welcher zum Zweck einer Blutbehandlung eines Patienten mit einer Blutbehandlungsvorrichtung verbindbar oder verbunden ist bzw. sind, oder jeweils aus Abschnitten hiervon.

Das offenbarte Verfahren umfasst das Fördern des im extrakorporalen Blutkreislauf nach Beenden der Blutbehandlungssitzung noch vorliegenden Fluids mittels einer Blutpumpe, welche bereits zur Blutbehandlung verwendet wurde. Während die Blutpumpe bei der Blutbehandlung in einer ersten Förderrichtung gefördert hat, wird mittels der Blutpumpe beim offenbarten Verfahren in einer zweiten, der ersten Förderrichtung entgegengesetzten Förderrichtung gefördert. Zur Durchführung des offenbarten Verfahrens wird oder ist ein erster Abschnitt des arteriellen Leitungsabschnitts mit einem zweiten Abschnitt des venösen Leitungsabschnitts des extrakorporalen Blutkreislaufs in Fluidverbindung verbunden.

Dabei ist oder wird in bestimmten Ausführungsformen die Förderrate der Blutpumpe eingestellt, um zu wenigstens einem Zeitpunkt oder während wenigstens eines Zeitraums, dauerhaft und/oder bis zum Erreichen eines vorbestimmten maximalen Wertes der Förderrate, auf welchen die Förderrate der Blutpumpe begrenzt ist, zwischen 75 % und 90 %, vorzugsweise zwischen 80 % und 84 %, besonders bevorzugt 82 % einer Förderrate einer zweiten Fördereinrichtung der verwendeten Blutbehandlungsvorrichtung zu betragen. Dies kann optional vorbehaltlich unter Berücksichtigung einer voreingestellten Begrenzung erfolgen.

Die zur Durchführung des offenbarten Verfahrens verwendete Blutbehandlungsvorrichtung weist neben der Blutpumpe wenigstens eine zweite Fördereinrichtung, insbesondere eine Substituatpumpe, zum Einbringen eines zweiten Fluids, insbesondere einer Substituatflüssigkeit, in das Leitungsinnere des extrakorporalen Blutkreislaufs und/oder zum Fördern eines Leitungsinhalts innerhalb des Leitungsinneren des extrakorporalen Blutkreislaufs auf.

Die erfindungsgemäße Blutbehandlungsvorrichtung ist vorgesehen und ausgestaltet und/oder ausgestattet zur Durchführung des offenbarten Verfahrens.

Die erfindungsgemäße Steuereinrichtung ist geeignet und vorgesehen und/oder ausgelegt und/oder konfiguriert zur Durchführung des offenbarten Verfahrens im Zusammenwirken mit den hierzu jeweils erforderlichen Einrichtungen, beispielsweise wie im Folgenden beschrieben.

Ein erfindungsgemäßes digitales, insbesondere nichtflüchtiges, Speichermedium, insbesondere in Form eines maschinenlesbaren Trägers, insbesondere in Form einer Diskette, CD, EPROM oder DVD, mit elektrisch auslesbaren Steuersignalen kann derart mit einem programmierbaren Computersystem zusammenwirken, dass die maschinellen Schritte des offenbarten Verfahrens veranlasst werden.

Dabei können alle, einige oder manche der maschinell durchgeführten Schritte des offenbarten Verfahrens veranlasst werden.

Ein erfindungsgemäßes Computerprogramm-Produkt weist einen volatilen, flüchtigen oder auf einem maschinenlesbaren Träger gespeicherten Programmcode zur Veranlassung der maschinellen Schritte des offenbarten Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein erfindungsgemäßes Computerprogramm weist einen Programmcode auf zur Veranlassung der maschinellen Schritte des offenbarten Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden. Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

In einigen erfindungsgemäßen Ausführungsformen ist eine Förderrate der Blutpumpe auf einen, insbesondere durch den Anwender, vorbestimmten Wert begrenzt, vorzugsweise auf einen Wert zwischen 90 und 110 ml/min.

Der extrakorporale Blutkreislauf ist in bestimmten Ausführungsformen der vorliegenden Erfindung ein Schlauchset. In jedem Fall ist der extrakorporale Blutkreislauf vorgesehen zum extrakorporalen Leiten von Blut eines Patienten, beispielsweise bei der Hämodialyse, der Hämofiltration, der Hämodiafiltration oder dergleichen.

In einigen erfindungsgemäßen Ausführungsformen ist der extrakorporale Blutkreislauf zumindest abschnittsweise als integraler und ggf. unlösbarer Bestand der Funktionseinrichtung ausgestaltet, in anderen nicht. So kann sich ein frei beweglicher Schlauchabschnitt des extrakorporalen Blutkreislauf einstückig oder integral auf oder in der Funktionseinrichtung, beispielsweise einer Blutkassette, fortsetzen und umgekehrt.

Eine Funktionseinrichtung ist in bestimmten Ausführungsformen der vorliegenden Erfindung eine Einrichtung, welche bei einer Blutbehandlung verwendet wird. Beispiele für Funktionseinrichtungen schließen interne und externe Funktionseinrichtungen, medizinische Disposables, insbesondere Blutkassetten wie eine Einmal-Blutkassette, oder andere blutführende Einrichtungen ein.

Beispielhafte Ausführungsformen einer Blutkassette sind insbesondere in der Anmeldung der Anmelderin mit der Veröffentlichungsnummer DE 10 2009 018 664 A1 mit dem Titel *"Externe Funktionseinrichtung, Blutbehandlungsvorrichtung zum Aufnehmen einer erfindungsgemäßen externen Funktionseinrichtung, sowie Verfahren",* die am 23.04.2009 beim Deutschen Patent- und Markenamt eingereicht wurde, und in der Anmeldung der Anmelderin mit der Veröffentlichungsnummer DE 10 2009 024 468 A1 mit demselben Titel, die am 10.06.2009 beim Deutschen Patent- und Markenamt eingereicht wurde, offenbart.

In bestimmten erfindungsgemäßen Ausführungsformen ist die Funktionseinrichtung als externe Funktionseinrichtung kein Teil der Blutbehandlungsvorrichtung, d h. kein integrales Bauteil derselben. In anderen Ausführungsformen kann die Funktionseinrichtung Teil der Blutbehandlungsvorrichtung sein.

Eine Blutbehandlungsvorrichtung ist dazu vorgesehen und/oder ausgestaltet, eine medizinische Behandlung, insbesondere eine Blutbehandlung des Patienten, z. B. eine Dialyse, auszuführen oder zu veranlassen. Hierzu ist die Blutbehandlungsvorrichtung mit wenigstens einem extrakorporalen Blutkreislauf, welcher ein Leitungsinneres aufweist, verbunden oder weist einen solchen auf.

Der arterielle Leitungsabschnitt des extrakorporalen Blutkreislaufs ist in bestimmten Ausführungsformen der vorliegenden Erfindung jener Leitungsabschnitt, in welchen das den Patientenkörper zum Zwecke der extrakorporalen Blutbehandlung verlassende Patientenblut einströmt und in welchem es sich vor Eintritt in die Blutbehandlungseinrichtung, z. B. einen Dialysator, befindet.

In bestimmten Ausführungsformen der vorliegenden Erfindung ist oder umfasst der erste Abschnitt des arteriellen Leitungsabschnitts den arteriellen Nadelanschluss zum Patienten, z. B. den arteriellen Nadelanschluss bei einem Double-Needle-Dialyseverfahren.

Der venöse Leitungsabschnitt des extrakorporalen Blutkreislaufs ist in manchen Ausführungsformen der vorliegenden Erfindung jener Leitungsabschnitt, aus welchem das extrakorporal behandelte Patientenblut nach seiner Behandlung in einer Blutbehandlungseinrichtung, beispielsweise einem Dialysator, zum Patientenkörper hin oder in diesen zurückströmt.

In bestimmten Ausführungsformen der vorliegenden Erfindung ist oder umfasst der zweite Abschnitt des venösen Leitungsabschnitts einen venösen Port, beispielsweise einen venösen Zugabeport oder eine venöse Zugabestelle. Dieser kann ggf. auch genutzt werden oder vorgesehen sein zur Zugabe von Substituatflüssigkeit, Calciumcitrat, Heparin oder dergleichen zu dem im venösen Leitungsabschnitt des extrakorporalen Blutkreislaufs strömenden Patientenblut.

In bestimmten Ausführungsformen der vorliegenden Erfindung mündet die venöse Zugabestelle des venösen Leitungsabschnitts des extrakorporalen Blutkreislaufs stromaufwärts einer Blutkammer und stromaufwärts eines Gerinnselfängers, optional direkt oder indirekt, in den venösen Leitungsabschnitt.

In manchen erfindungsgemäßen Ausführungsformen ist die venöse Zugabestelle integraler Teil einer Blutkassette.

Die Blutbehandlungsvorrichtung weist eine Blutpumpe zum Fördern von Blut innerhalb des Leitungsinneren des extrakorporalen Blutkreislaufs auf.

In bestimmten Ausführungsformen der vorliegenden Erfindung entspricht die - während der Blutbehandlung übliche - erste Förderrichtung einer Förderrichtung vom arteriellen Zugang (Blutentnahmestelle) vom Patienten zu einer Blutbehandlungseinrichtung, beispielsweise einem Blutfilter oder einem Dialysator, und anschließend durch den venösen Leitungsabschnitt des extrakorporalen Blutkreislaufs zum venösen Zugang (Blutrückgabestelle).

Die der ersten Förderrichtung entgegengesetzte zweite Förderrichtung verläuft daher in solchen Ausführungsformen von venös nach arteriell.

Ein "Entfernen von Blut" bezeichnet in bestimmten Ausführungsformen der vorliegenden Erfindung ein vollständiges - oder im Wesentlichen oder nahezu vollständiges - oder ein teilweises Entfernen von Blut aus dem extrakorporalen Blutkreislauf nach Beenden einer Blutbehandlungssitzung.

Ein Reinfundieren des aus dem extrakorporalen Blutkreislauf entfernten Bluts in das Gefäßsystem des Patienten selbst ist in einigen Ausführungsformen der vorliegenden Erfindung nicht Teil des offenbarten Verfahrens, in anderen hingegen schon.

Die Blutbehandlungsvorrichtung weist wenigstens eine zweite Fördereinrichtung auf. Die zweite Fördereinrichtung dient dem Einbringen wenigstens eines zweiten, vom Blut verschiedenen Fluids, beispielsweise einer Substituatflüssigkeit, in das Leitungsinnere des extrakorporalen Blutkreislaufs und/oder zum Fördern des Fluids darin.

Das Einbringen des zweiten Fluids, im Folgenden vereinfachend - aber nicht einschränkend - als Substituatflüssigkeit bezeichnet, in das Leitungsinnere des extrakorporalen Blutkreislaufs durch Betreiben der zweiten Fördereinrichtung, im Folgenden vereinfachend - aber nicht einschränkend - als Substituatpumpe bezeichnet, erfolgt in bestimmten Ausführungsformen der vorliegenden Erfindung nach Verbinden des ersten Abschnitts des arteriellen Leitungsabschnitts mit dem zweiten Abschnitt des venösen Leitungsabschnitts.

In manchen erfindungsgemäßen Ausführungsformen wird die Blutpumpe derart in der zweiten Förderrichtung betrieben, dass der in das Leitungsinnere des extrakorporalen Blutkreislaufs eingebrachte Substituatflüssigkeitsstrom aufgeteilt wird in wenigstens einen ersten und einen zweiten Teilstrom. Der erste Substituatflüssigkeitsteilstrom bewegt sich zu einer Blutbehandlungseinrichtung hin, der zweite Substituatflüssigkeitsteilstrom bewegt sich in der zweiten Förderrichtung.

In bestimmten Ausführungsformen der vorliegenden Erfindung umfasst das offenbarte Verfahren das Dekonnektieren des ersten Abschnitts.

In manchen Ausführungsformen der vorliegenden Erfindung umfasst das offenbarte Verfahren das Konnektieren des ersten Abschnitts mit einer venösen Zugabestelle des venösen Leitungsabschnitts des extrakorporalen Blutkreislaufs oder mit einer venösen Zugabestelle der Funktionseinrichtung (z. B. der Blutkassette).

In bestimmten Ausführungsformen der vorliegenden Erfindung werden die zweite Fördereinrichtung und die Blutpumpe, insbesondere im Wesentlichen oder nahezu oder vollständig, zeitgleich gestartet.

Die Förderraten der zweiten Fördereinrichtung und der Blutpumpe werden oder sind in manchen Ausführungsformen der vorliegenden Erfindung derart eingestellt, dass ein Abschnitt des extrakorporalen Blutkreislaufs, z. B. von einer Prädilutionsstelle oder von einem Prädilutionsventil bis zur venösen Zugabestelle oder bis zu einer Stelle, an welcher sich Fluid aus dem venösen Leitungsabschnitt und Fluid aus dem arteriellen Leitungsabschnitt vereinen oder aufeinandertreffen, nicht eher von Blut geleert wird als ein Abschnitt des arteriellen Leitungsabschnitts, z. B. bis zu dessen Konnektion mit der venösen Zugabestelle oder bis zu jener Stelle, an welcher sich Fluid aus dem venösen Leitungsabschnitt mit Fluid aus dem arteriellen Leitungsabschnitt vereint oder beide Fluide aufeinandertreffen.

In bestimmten Ausführungsformen der vorliegenden Erfindung ist die Förderrate der Blutpumpe (anfangs, stets, oder im Mittel) niedriger als die Förderrate der zweiten Fördereinrichtung oder wird entsprechend eingestellt.

In manchen Ausführungsformen der vorliegenden Erfindung werden die Förderraten der Blutpumpe und/oder der zweiten Fördereinrichtung während des Entfernens von Blut mittels Drucküberwachung und/oder Druckmessung und/oder Druckbegrenzung überwacht und/oder geregelt.

In einigen Ausführungsformen der vorliegenden Erfindung werden die Förderraten der Blutpumpe und der zweiten Fördereinrichtung während des Entfernens von Blut mittels Drucküberwachung und/oder Druckmessung und/oder Druckbegrenzung überwacht und/oder geregelt, insbesondere mittels mehrfachen Messens eines aktuellen Förderdrucks der Blutpumpe, z. B. in zeitlichem Zusammenhang mit dem Eingriff oder der Stellung oder des Drehwinkels einer Rolle der Blutpumpe, sofern diese als Rollenpumpe ausgestaltet ist, Ermittelns einer Regeldifferenz zwischen gemessenem Förderdruck der Blutpumpe und einem Zielwert, und Anhebens der Förderrate der Blutpumpe entsprechend der Regeldifferenz.

In einigen Ausführungsformen der vorliegenden Erfindung wird der aktuelle Förderdruck bei Erreichen eines vorbestimmten Drehwinkels oder Drehwinkelbereichs eines Pumprotors der Blutpumpe abgegriffen, gemessen oder errechnet.

In manchen Ausführungsformen der vorliegenden Erfindung wird der aktuelle Förderdruck mittels des arteriellen Drucksensors gemessen.

In einigen Ausführungsformen der vorliegenden Erfindung werden die Förderrate der Blutpumpe und/oder die Förderrate der zweiten Fördereinrichtung mit der Absicht eingestellt oder begrenzt, um einen Zieldruck P_art,soll zu erreichen und/oder nicht zu übersteigen. Der Zieldruck ermittelt sich gemäß der Formel (I):

$$\text{P\_art,soll} = a*\text{P\_art,max}$$

wobei gilt:

P_art,soll      ist der mittels arteriellem Druckmesser gemessene Druck;

a      ist eine Konstante; sie liegt bevorzugt zwischen 0,85 und 0,95, besonders bevorzugt beträgt sie 0,9; und

P_art,max      ist eine voreingestellte oder vorbestimmte Messbereichsgrenze oder ein zulässiger Maximalwert für den mittels des arteriellen Druckmessers gemessenen Drucks und ist vorzugsweise eine Alarmauslösegrenze.

In einigen Ausführungsformen der vorliegenden Erfindung umfasst das offenbarte Verfahren das Erhöhen der Förderrate der zweiten Fördereinrichtung gemäß der Formel (II):

$$\text{Q5000\_künftig} = \text{Q5000\_aktuell} + ((\text{P\_art,soll})-(\text{P\_art,scan}))*b$$

Dabei gilt:

Q5000_künftig      ist eine jeweils künftige, mittels der Formel (II) zu einem oder für einen Zeitpunkt t_künftig festzusetzende Förderrate der zweiten Fördereinrichtung;

Q5000_aktuell      ist eine aktuelle Förderrate der zweiten Fördereinrichtung zu einem Zeitpunkt t_aktuell, welche durch Q5000_künftig abzulösen ist und dieser zeitlich jeweils vorausgeht, wobei t_aktuell vor t_künftig liegt; t_künftig könnte auch als t(x+1) und t_aktuell als t(x) bezeichnet werden;

P_art,soll      ist ein voreingestellter oder ermittelter Zielwert, welcher vom arteriellen Druckmesser als Zielwert gemessen werden soll; er entspricht damit einem Soll-Wert;

P_art,scan      ist ein Druckwert, welcher am arteriellen Druckmesser während des Anliegens der aktuellen Förderrate Q5000_aktuell der zweiten Fördereinrichtung gemessen wird; er entspricht damit einem Ist-Wert oder einem Wert P_art,aktuell; und

b ist eine Konstante; sie liegt bevorzugt zwischen 0,10 und 0,15, besonders bevorzugt beträgt sie 0,12.

In bestimmten Ausführungsformen der vorliegenden Erfindung umfasst das offenbarte Verfahren das Senken der Förderrate der zweiten Fördereinrichtung gemäß der Formel (III):

$$Q5000\_künftig = Q5000\_aktuell + ((P\_art,soll)-(P\_art,scan))*c$$

wobei gilt:

Q5000_künftig ist eine jeweils künftige, festzusetzende Förderrate der zweiten Fördereinrichtung zu einem Zeitpunkt t_künftig;

Q5000_aktuell ist eine aktuelle Förderrate der zweiten Fördereinrichtung zu einem Zeitpunkt t_aktuell, welche durch Q5000_künftig abzulösen ist und dieser zeitlich jeweils vorausgeht, wobei t_aktuell vor t_künftig liegt; t_künftig könnte auch als t(x+1) und t_aktuell als t(x) bezeichnet werden;

P_art,soll ist ein voreingestellter Zielwert, welcher am arteriellen Druckmesser anliegen soll;

P_art,scan ist ein Druckwert, welcher am arteriellen Druckmesser während des Anliegens der aktuellen Förderrate_Q5000_aktuell gemessen wird; und

c ist eine Konstante; sie liegt bevorzugt zwischen 0,20 und 0,30, besonders bevorzugt beträgt sie 0,24.

In bestimmten Ausführungsformen der vorliegenden Erfindung wird die Förderrate der zweiten Fördereinrichtung derart eingestellt, dass an der venösen Zugabestelle - oder an einer Stelle, an welcher Fluid aus dem venösen Leitungsabschnitt mit Fluid aus dem arteriellen Leitungsabschnitt vereint wird oder auf dieses trifft - Blut (aus einer Leitung) und Substituatflüssigkeit (aus einer anderen Leitung) gleichen Verdünnungsgrads aufeinandertreffen.

In bestimmten Ausführungsformen des offenbarten Verfahrens ist vorgesehen, die Blutpumpe zu stoppen oder ihre Fördergeschwindigkeit oder -rate zu reduzieren, bevor Substituatflüssigkeit stromab (bezogen auf die zweite Förderrichtung oder auf das offenbarte Verfahren) der venösen Zugabestelle oder der Stelle, an welcher sich Fluid aus dem venösen Leitungsabschnitt und Fluid aus dem arteriellen Leitungsabschnitt vereinen oder aufeinander treffen, gelangt.

Dies kann in bestimmten Ausführungsformen der vorliegenden Erfindung vorteilhaft zu einem stärkeren und/oder verbesserten Durchspülen der Blutbehandlungseinrichtung beitragen.

Der Zeitpunkt zum Stoppen der Blutpumpe oder zum Reduzieren ihrer Förderrate wird in bestimmten Ausführungsformen der vorliegenden Erfindung mit Hilfe des in den extrakorporalen Blutkreislauf integrierten arteriellen Luftblasendetektors/optischen Detektors (auch bekannt als air bubble detector/optical detector, ABD/OD) bestimmt. Dies kann vorteilhaft die Genauigkeit, mit welcher der Zeitpunkt zum Stoppen ermittelt wird, erhöhen.

In anderen Ausführungsformen der vorliegenden Erfindung umfasst das offenbarte Verfahren das Stoppen der zweiten Fördereinrichtung oder das Reduzieren der Geschwindigkeit oder Förderrate der zweiten Fördereinrichtung, bevor Substituatflüssigkeit stromab (bezogen auf die zweite Förderrichtung oder auf das offenbarte Verfahren) der venösen Zugabestelle oder der Stelle, an welcher sich Fluid aus dem venösen Leitungsabschnitt und Fluid aus dem arteriellen Leitungsabschnitt vereinen oder aufeinander treffen, gelangt. Dies kann vorteilhaft zu einer stärkeren und/oder verbesserten Durchspülung des arteriellen Leitungsabschnitts des extrakorporalen Blutkreislaufs beitragen. Die Blutpumpe wird in solchen Ausführungsformen weiter betrieben, oder nicht.

In bestimmten Ausführungsformen der vorliegenden Erfindung umfasst das offenbarte Verfahren das Überprüfen der Konnektion des ersten Abschnitts, z. B. des arteriellen Nadelanschlusses, des extrakorporalen Blutkreislaufs an der venösen Zugabestelle des venösen Leitungsabschnitts des extrakorporalen Blutkreislaufs (Konnektionstest).

In bestimmten Ausführungsformen der vorliegenden Erfindung umfasst das Überprüfen das Erzeugen eines Druckausgleichs.

In solchen Ausführungsformen kann vorgesehen sein, die Blutpumpe und/oder die zweite Fördereinrichtung zu stoppen. Ferner kann vorgesehen sein, die arterielle Klemme zu öffnen oder offen zu halten.

In bestimmten weiteren Ausführungsformen der vorliegenden Erfindung umfasst die Überprüfung das Bestimmen eines diastolischen Patientendrucks. Ein Minimalwert des diastolischen Patientendrucks kann über einen Zeitraum von z. B. 2,5 s gespeichert werden. Anschließend wird mittels der in der ersten Förderrichtung, also vorwärts, fördernden Blutpumpe ein Unterdruck erzeugt, wobei die venöse Klemme geöffnet wird oder bereits offen steht. In bestimmten Ausführungsformen der vorliegenden Erfindung ist definiert, dass der Gefäßdruck des Patienten innerhalb eines Zeitraums von 2,1 s um 50 mmHg abfallen muss, damit der Konnektionstest als bestanden gilt. Alternativ können andere Werte zu Druckabfall und/oder Zeiten als die hier genannten beachtet werden. Sollte die gewünschte und/oder erforderliche Druckreduktion nicht oder nicht in der vorbestimmten Zeit erfolgt sein, kann der Konnektionstest als nicht bestanden gelten.

In manchen erfindungsgemäßen Ausführungsformen umfasst das offenbarte Verfahren einen Schritt des Freispülens des Prädilutionsventils. Hierzu wird die Förderrate der zweiten Fördereinrichtung, beispielsweise bolusartig, beispielsweise kurzfristig, von vorzugsweise 0 ml/min auf einen vorbestimmten Wert erhöht. Dieser kann optional 100 ml/min betragen. Ergänzend oder alternativ zum Fördern mit dem vorgenannten vorbestimmten Wert, wird während des Freispülens eine vorbestimmte Fördermenge gefördert. Diese beträgt beispielsweise 10 ml. Das Freispülen erfolgt vorzugsweise ausgehend von 0 ml/min. Nach Erreichen des vorbestimmten Wertes der Förderrate und/oder nach Fördern des vorbestimmten Volumens kann die Förderrate optional wieder auf 0 ml/min abfallen.

Die erfindungsgemäße Funktionseinrichtung ist in einigen erfindungsgemäßen Ausführungsformen als Disposable oder als Wegwerfartikel ausgestaltet.

In bestimmten erfindungsgemäßen Ausführungsformen ist die venöse Zugabestelle der Funktionseinrichtung ausgestaltet, um durch einfaches Verklemmen, Aufstecken oder Verschrauben eines arteriellen Nadelanschlusses oder eines anderen Abschnitts des arteriellen Leitungsabschnitts des extrakorporalen Blutkreislaufs hiermit eine Fluidverbindung zu erzeugen. Eine entsprechende Ausgestaltung und/oder entsprechende Einrichtungen können erfindungsgemäß vorgesehen sein.

In manchen erfindungsgemäßen Ausführungsformen sind die venöse Zugabestelle der Funktionseinrichtung und der arterielle Nadelanschluss oder ein anderer Abschnitt des arteriellen Leitungsabschnitts des extrakorporalen Blutkreislaufs vom selben Konnektorsystem, beispielsweise beide Luer-Konnektoren, oder weibliches und männliches Verbindungsstück.

In gewissen erfindungsgemäßen Ausführungsformen ist die Anschlussgeometrie der venösen Zugabestelle gleich oder entsprechend der Anschlussgeometrie einer arteriellen Nadel, mit welcher der extrakorporale Blutkreislauf zu seiner Benutzung verbunden wird, ausgestaltet.

Die erfindungsgemäße Steuereinrichtung ist in einigen erfindungsgemäßen Ausführungsformen als Regeleinrichtung ausgestaltet.

Die erfindungsgemäße Blutbehandlungseinrichtung ist in manchen erfindungsgemäßen Ausführungsformen als Hämodialysevorrichtung oder Hämofiltrationsvorrichtung oder Hämodiafiltrationsvorrichtung ausgestaltet.

Die erfindungsgemäße Blutbehandlungseinrichtung weist in bestimmten erfindungsgemäßen Ausführungsformen wenigstens eine erfindungsgemäße Steuereinrichtung auf.

Manche oder alle erfindungsgemäßen Ausführungsformen können einen, mehrere oder alle der oben und/oder im Folgenden genannten Vorteile aufweisen.

Die vorliegende Erfindung stellt ein einfaches und wenig aufwendiges Verfahren zum Entfernen von Blut aus einem extrakorporalen Blutkreislauf nach einer Blutbehandlungssitzung sowie entsprechende Vorrichtungen bereit. Durch das Entfernen von Blut nach Behandlungsende kann die Gefahr einer Kontaminierung bei der weiteren Handhabung oder Entsorgung des Blutkreislaufs vorteilhaft verringert werden.

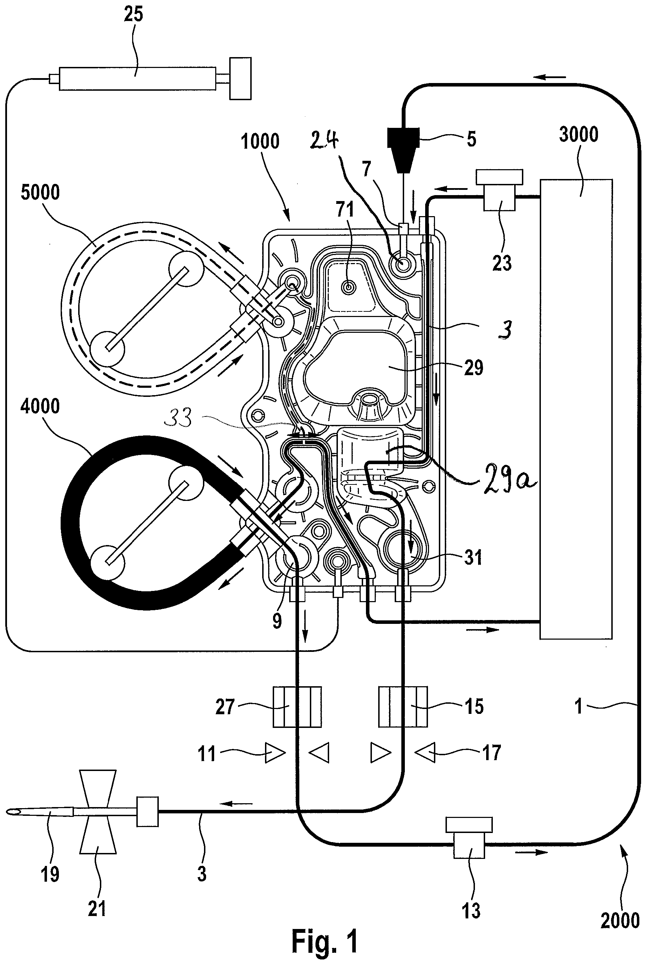

Während einer Blutbehandlung könnten sich an konstruktionsbedingten Unebenheiten im Bereich des Prädilutionsventils (siehe Bezugszeichen 33 in Fig. 1) Blutgerinnsel bilden. Während eines höchst vorsorglichen Freispülens, wie hierin beschrieben, fördert die zweite Fördereinrichtung solche Blutgerinnsel mit einer vorgegebenen Freispülrate, beispielsweise unter Einbringen von etwa 10 ml Volumen, vom Prädilutionsventil in Richtung Eingang des Blutfilters. Hierbei wird das Prädilutionsventil von Blutgerinnseln befreit. Aufgrund des hierin beschriebenen Verhältnisses zwischen der Förderrate der Blutpumpe und der Förderrate der zweiten Fördereinrichtung (vorzugsweise 82%) ist vorteilhaft sichergestellt, dass solche Blutgerinnsel nicht in Richtung des venösen Leitungsabschnitts bis zur venösen Nadel verbracht werden, sondern sich an der Bluteingangsseite des Blutfilters ablagern, von wo aus sie bei Anlegen des vorgenannten Verhältnisses zwischen der Förderrate der Blutpumpe und der Förderrate der zweiten Fördereinrichtung (vorzugsweise 82%) nicht weiter stromauf bewegt werden und folglich nicht zum venösen Leitungsabschnitt oder gar zur venösen Nadel und hierüber in das Gefäßsystem des Patienten gelangen können. Dieser vorteilhafte Umstand ist mit anderen oder deutlich anderen Verhältnissen nicht erzielbar. Ferner gilt, dass selbst einem aufgrund eines Anwenderfehlers irrtümlich während der Reinfusion noch mit der arteriellen Nadel verbundenen Patienten somit vorteilhaft kein Blutgerinnsel infundiert werden kann. Ferner können auch die venöse Zugabestelle und das Rückschlagventil vorteilhaft nicht durch solche Blutgerinnsel verstopft werden, was zu einem raschen und störungsunanfälligen Ablauf beiträgt.

Der Anwender kann in bestimmten erfindungsgemäßen Ausführungsformen den für die Entfernung des Bluts aus einem extrakorporalen Blutkreislauf zulässigen, gewünschten, oder verschriebenen Maximalfluss an der Blutbehandlungsvorrichtung einstellen und muss keine weiteren Eingaben tätigen.

In einigen erfindungsgemäßen Ausführungsformen ist aufgrund der vorgeschlagenen Regelung sichergestellt, dass es zu keinen Messbereichsüberschreitungen am arteriellen Drucksensor kommt. Hierdurch werden dem Patienten und dem Anwender vermeidbare Unterbrechungen der Behandlung erspart.

In gewissen erfindungsgemäßen Ausführungsformen wird eine größtmögliche Förderrate über den arteriellen Leitungsabschnitt und/oder die Blutbehandlungsvorrichtung gefördert, dies trägt dazu bei, eine Sedimentation (und somit auch die im Blutfilter und/oder extrakorporalen Blutkreislauf verbleibende Restblutmenge) gering zu halten.

In einigen erfindungsgemäßen Ausführungsformen ist eine größtmögliche Förderrate der zweiten Fördereinrichtung sichergestellt, mittels welcher das Blut aus der gesamten Blutbehandlungsvorrichtung schnellstmöglich entfernt werden kann.

In gewissen erfindungsgemäßen Ausführungsformen ist eine dynamische Steuerung der Förderraten von der Blutpumpe sowie der zweiten Fördereinrichtung je nach Ausgangs-Viskosität des Blutes und abnehmender Viskosität des zunehmend verdünnten Blutes während der Reinfusion aufgrund der erfindungsgemäß vorgeschlagenen Drucksteuerung vorteilhaft möglich. Die Dynamik oder die erfindungsgemäße Berücksichtigung der Dynamik ergibt sich aus der oder besteht in der Viskosität des Bluts, welche sich inter-individuell, also zwischen verschiedenen Patienten, aber auch intra-individuell, also bei ein und demselben Patienten, aber zu verschiedenen Zeitpunkten und sogar während einer Reinfusion oder Entleerung des Blutschlauchs, verändern kann.

Der arterielle Leitungsabschnitt (siehe Bezugszeichen 1 in Fig. 1) weist im Bereich der venösen Zugabestelle (Bezugszeichen 7 in Fig. 1), mit welcher er zur Reinfusion von Blut und/oder zum Leeren des Blutfilters oder des extrakorporalen Blutkreislaufs verbunden ist, aus funktionellen oder konstruktiven Vorgaben einen vergleichsweise geringen Querschnitt auf. Den geringen Querschnitt benötigt er, damit über ihn - und insbesondere über die venöse Zugabestelle - bestimmungsgemäß eine dosierte Gabe von Medikamenten (typischerweise z. B. Q < 1ml/min) appliziert werden kann. Aufgrund des geringen Querschnitts der venösen Zugabestelle hat diese im Vergleich zum arteriellen oder zum venösen Leitungsabschnitt einen vergleichsweise hohen Flusswiderstand. Daher ist Verwendung der Vorrichtung beispielsweise nach WO 2013/017240 A2 zur Reinfusion unkomfortabel und/oder physiologisch ungünstig. Denn möchte der Anwender vermeiden, dass der Druck am Drucksensor bis an dessen obere Messbereichsgrenze steigt, was zu einer Alarmierung des Anwenders und einer Behandlungsunterbrechung führen würde, so stellt der Anwender in der Praxis eine entsprechend niedrige Förderrate (typischerweise 70 ml/min) für die zweite Fördereinrichtung ein. Die niedrige Förderrate hat nachteilig eine geringe Effizienz der Blutrückgabe zur Folge, da zu Abschluss der Reinfusion oder Entleerung der Blutbehandlungseinrichtung, z. B. des Blutfilters, mehr Restblut in der Behandlungsvorrichtung verbleibt. Eine weitere nachteilige Folge der niedrigen Förderrate ist eine Sedimentation des Blutes innerhalb der Blutbehandlungsreinrichtung, was wiederum zu einer geringeren Effizienz der Blutrückgabe führt, da zu Abschluss der Reinfusion oder Entleerung der Blutbehandlungseinrichtung, z. B. des Blutfilters, mehr Restblut in der Behandlungseinrichtung verbleibt. Eine nachteilige Folge der niedrigen Förderrate ist auch, dass die Reinfusionsprozedur aufgrund der geringe Förderraten lang dauert. Diese nachteiligen Folgen treten bei manchen erfindungsgemäßen Ausführungsformen vorteilhafter Weise nicht auf.

Aber auch eine höhere Förderrate, vom Anwender eingestellt, hat beim Vorgehen des Standes der Technik nachteilige Folgen. Denn die höhere Förderrate (typischerweise 150 ml/min) führt erfahrungsgemäß regelmäßig zu Alarmierungen wegen Messbereichsüberschreitung am Drucksensor. Dies macht ein Eingreifen des Anwenders notwendig. Die Entleerungs- oder Reinfusionsprozedur dauert wegen der hierdurch bedingten Unterbrechungen länger, was wiederum zur Sedimentation im arteriellen Leitungsabschnitt und in der Blutbehandlungseinrichtung führt. Auch diese Nachteile des Standes der Technik sind erfindungsgemäß vermeidbar.

In bestimmten erfindungsgemäßen Ausführungsformen erlaubt das offenbarte Verfahren vorteilhaft, nach der Blutbehandlungssitzung im extrakorporalen Blutkreislauf vorhandenes Blut über eine venöse Verbindung mit dem Gefäßsystem des Patienten vollständig an diesen zurückzugeben. Dabei ist es vermeidbar, dem Patienten zugleich Substituatflüssigkeit oder ein anderes Fluid zu infundieren.

Das offenbarte Verfahren lässt sich in einigen erfindungsgemäßen Ausführungsformen bei aus der Praxis bekannten Behandlungsvorrichtungen vorteilhaft bereits allein mittels eines einfach durchzuführenden Software-Updates umsetzen. Die erforderlichen Maschinenelemente weisen aus der Praxis bekannte Blutbehandlungsvorrichtungen häufig bereits auf.

Im Folgenden wird die vorliegende Erfindung unter Bezugnahme auf die beigefügten Figuren rein exemplarisch beschrieben. Es gilt:

Fig. 1    zeigt in vereinfachter Darstellung eine medizinische Funktionseinrichtung mit einem extrakorporalen Blutkreislauf, welcher mittels des offenbarten Verfahrens von Blut befreit werden kann; und

Fig. 2    zeigt Druck- und Volumenverläufe über der Zeit, welche beim Ausführen einer beispielhaften Ausführungsform des offenbarten Verfahrens mittels der in Fig. 1 gezeigten Anordnung auftreten oder gemessen werden können.

Fig. 1 zeigt schematisch vereinfacht eine Funktionseinrichtung 1000 mit einem hieran angeschlossenen extrakorporalen Blutkreislauf 2000.

Der extrakorporale Blutkreislauf 2000 weist eine Blutbehandlungseinrichtung 3000, z. B. einen Blutfilter oder Dialysator, auf oder ist mit dieser verbunden.

Eine in Fig. 1 nur durch einige ihrer Einrichtungen repräsentierte Blutbehandlungsvorrichtung, mittels welcher das hier beschriebene Verfahren ganz oder im Wesentlichen mit oder unter einem Reinfundieren des im extrakorporalen Blutkreislauf 2000 enthaltenen Bluts automatisiert abläuft, weist eine Blutpumpe 4000 und eine zweite Fördereinrichtung 5000 auf. Sowohl die Blutpumpe 4000 als auch die zweite Fördereinrichtung 5000 fördern Fluid durch Abschnitte der Funktionseinrichtung 1000 und des extrakorporalen Blutkreislaufs 2000. Die Funktionseinrichtung 1000 ist beispielhaft eine Blutkassette.

Angedeutet sind ein Anschlussschema sowie Flussrichtungen, angegeben durch Pfeile, von Blut und Substituat (als Beispiel eines zweiten Fluids) während der Entfernung des Bluts mit gleichzeitiger Reinfusion des Bluts. Der einzige Doppelpfeil beschreibt eine Aufteilung des Substituatstroms in zwei Teilströme.

Der extrakorporale Blutkreislauf 2000 weist einen arteriellen Leitungsabschnitt 1 sowie einen venösen Leitungsabschnitt 3 auf.

Der arterielle Leitungsabschnitt 1 weist einen ersten Abschnitt auf. Der erste Abschnitt ist im Beispiel der Fig. 1 beispielhaft als arterieller Nadelanschluss 5 ausgestaltet.

Der venöse Leitungsabschnitt 3 weist einen zweiten Abschnitt auf. Der zweite Abschnitt 3 ist in Fig. 1 beispielhaft als venöse Zugabestelle 7 der Funktionseinrichtung 1000 ausgestaltet.

Der arterielle Leitungsabschnitt 1 weist einen arteriellen Drucksensor auf, welcher an der mit Bezugszeichen 9 bezeichneten Stelle an die Funktionseinrichtung 1000 gekoppelt ist ohne selbst Bestandteil der Funktionseinrichtung 1000 zu sein. Dieser Drucksensor dient u. a. zum Messen des Drucks oder zur Bestimmung des Druckabfalls während eines optionalen Konnektionstests. Er ist im arteriellen Leitungsabschnitt 1 des extrakorporalen Blutkreislaufs 2000 angeordnet.

Der arterielle Leitungsabschnitt 1 weist eine arterielle Klemme 11 auf.

Der arterielle Leitungsabschnitt 1 weist ein arterielles Septum 13, hier optional in Gestalt einer Zugabeeinrichtung, auf.

Der venöse Leitungsabschnitt 3 weist einen venösen Luftblasendetektor/optischen Sensor 15 auf.

Der venöse Leitungsabschnitt 3 weist eine venöse Klemme 17 auf.

Der venöse Leitungsabschnitt 3 weist eine venöse Nadel 19 an einem venösen Patientenkonnektor 21 auf.

Zwischen der Blutbehandlungseinrichtung 3000 und dem Ort seiner Verbindung mit dem auf der Funktionseinrichtung 1000 vorliegenden Abschnitt des extrakorporalen Blutkreislaufs 2000 ist ein venöses Septum 23, hier optional in Gestalt einer Zugabeeinrichtung, angeordnet.

Die Funktionseinrichtung 1000 weist ein Rückschlagventil 24 auf, welches eine Fluidzufuhr in der mittels Pfeil in Fig. 1 gezeigten Richtung über die venöse Zugabestelle 7 in die Funktionseinrichtung 1000 und in den venösen Leitungsabschnitt 3 hinein erlaubt, einen Fluss aus der venösen Leitungsabschnitt 3 über die venöse Zugabestelle 7 aus der Funktionseinrichtung 1000 heraus aber verhindert.

Zur Zugabe von Heparin in das Leitungsinnere des extrakorporalen Blutkreislaufs 2000 während einer extrakorporalen Blutbehandlung ist der extrakorporale Blutkreislauf 2000 über einen entsprechenden Port der Funktionseinrichtung 1000 mit einer Heparinspritze 25 verbunden.

Der arterielle Leitungsabschnitt 1 weist einen arteriellen Luftblasendetektor/optischen Sensor 27 auf.

Bei der Blutbehandlung wird der extrakorporale Blutkreislauf 2000, z. B. wie in Fig. 1 gezeigt in Form eines Schlauchsystems, über zwei Nadeln (im Falle einer Double-Needle-Dialyse), mit dem Gefäßsystem des Patienten verbunden. Zur Durchführung der Blutbehandlung wird der extrakorporale Blutkreislauf 2000 mit Blut des Patienten gefüllt und während der Behandlung durchströmt.

Im Folgenden wird ein erfindungsgemäßes Entfernen von Blut aus dem extrakorporalen Blutkreislauf unter Nutzung der venösen Zugabestelle 7 der Funktionseinrichtung 1000 beschrieben. In dem hier beschriebenen Beispiel einer maschinell durchgeführten Ausführungsform des Verfahrens erfolgt mit der Entfernung von Blut eine gleichzeitige Reinfusion dieses Bluts.

Das offenbarte Verfahren kann automatisch oder manuell durch den Arzt gestartet werden.

Hierzu wird in bestimmten Ausführungsformen der Erfindung nach Beendigung der Blutbehandlung der arterielle Patientenkonnektor vom arteriellen Nadelanschluss 5 dekonnektiert und mit einem Port der Blutkassette, hier die venöse Zugabestelle 7, in Fluidverbindung verbunden. Wie in der in Fig. 1 gezeigten Blutkassette zu sehen, welche rein optional eine Single-Needle-Kammer 29 aufweist, kann die venöse Zugabestelle 7 stromaufwärts einer Blutkammer 29a und eines Gerinnselfängers 31 direkt (in anderen Ausführungsformen aber auch indirekt) in den venösen Leitungsabschnitt 3 des extrakorporalen Blutkreislaufs 2000 münden.

Im Beispiel der Fig. 1 weist die Funktionseinrichtung 1000 im Bereich des Gerinnselfängers 31 oder nahe diesem, jedenfalls aber stromab hiervon, zugleich aber stromauf des venösen Luftblasensensors 15, einen in Fig. 1 nicht gezeigten venösen Drucksensor auf, welcher den Druck des vorbeiströmenden venösen Bluts oder Fluids messen kann. In anderen erfindungsgemäßen Ausführungsformen ist der venöse Drucksensor an anderer, geeigneter Stelle der Funktionseinrichtung 1000 oder des extrakorporalen Blutkreislaufs 2000 vorgesehen.

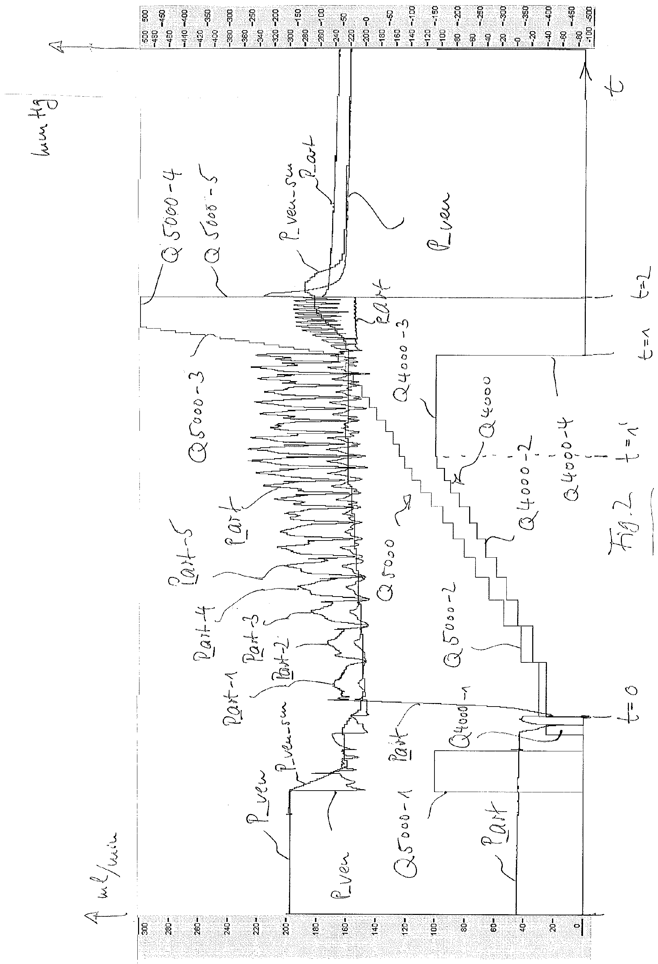

Die venöse Zugabestelle 7 ist in den o. g. Anmeldungen der Anmelderin mit den Veröffentlichungsnummern DE 10 2009 018 664 A1 und DE 10 2009 024 468 A1 jeweils in den Fig. 1 und 2 mit dem Bezugszeichen 37 gekennzeichnet. Die venöse Zugabestelle steht über das Rückschlagventil 24 mit der venösen Filterleitung in Verbindung.

Der Anwender kann zum Ende der Blutbehandlung aufgefordert werden, den arteriellen Nadelanschluss 5 vom arteriellen Patientenkonnektor (in Fig. 1 nicht gezeigt) zu trennen und den arteriellen Nadelanschluss 5 auf den Luer-Konnektor der venösen Zugabestelle 7 der Funktionseinrichtung 1000 zu schrauben oder auf andere Weise hiermit zu verbinden. Um zu prüfen, ob die Konnektion korrekt hergestellt ist, kann von der Blutbehandlungsvorrichtung automatisch oder auf Anforderung ein Konnektionstest durchgeführt werden. Dabei wird die korrekte Verbindung des arteriellen Leitungsabschnitts 1 mit dem venösen Leitungsabschnitt 3 geprüft. Ein direktes Fördern des Blutes über den arteriellen Leitungsabschnitt 1 in das Gefäßsystem des Patienten kann somit vorteilhaft vermieden werden.

Der venöse Leitungsabschnitt 3 ist in bestimmten Ausführungsformen mit dem optionalen Rückschlagventil 24 versehen, weshalb die Blutpumpe 4000 in der ersten Förderrichtung keine Flüssigkeit aus dem venösen Leitungsabschnitt 3 ansaugen kann. Daher darf erwartet werden, dass bei Durchführen des Konnektionstests der Druck im arteriellen Leitungsabschnitt 1 abnimmt. Nimmt der arterielle Druck wie erwartet ab, kann davon ausgegangen werden, dass der Patient arteriell nicht mehr konnektiert ist, jedenfalls aber die (manuelle) arterielle (Schlauch-)Klemme 11 geschlossen ist.

Ist, wie in bestimmten Ausführungsformen der vorliegenden Erfindung, ein arterieller Druckalarm bei Förderung in diesem Verfahrensstadium vorgesehen, so würde ein Fehler auch ohne Konnektionstest ebenfalls vorteilhaft frühzeitig erkannt werden. Daher kann es u. U. vorteilhaft möglich sein, auf ein explizites Testen des Konnektionszustands zu verzichten.

Der Konnektionstest zur Erkennung, ob der arterielle Leitungsabschnitt 1, z. B. mittels des arteriellen Nadelanschlusses 5, mit dem venösen Leitungsabschnitt 3, z. B. der venösen Zugabestelle 7, verbunden ist, kann dabei im Einzelnen folgendermaßen ablaufen: Zunächst wird ein Druckausgleich hergestellt, wobei die Blutpumpe 4000 und die zweite Fördereinrichtung 5000 gestoppt werden. Die arterielle Klemme 11 ist geöffnet.

Mittels des Drucksensors 9 wird der diastolische Patientendruck detektiert. Hierbei wird ein Minimalwert über beispielsweise 2,5 s gespeichert. Anschließend wird ein Unterdruck aufgebaut, wozu die venöse Klemme 17 geöffnet wird und mittels der Blutpumpe 4000 in der ersten Förderrichtung gefördert wird. Der Druck muss aufgrund des Rückschlagventils 24 innerhalb von beispielsweise 2,1 s um 50 mmHg unter den zuvor detektierten diastolischen Patientendruck abfallen, ansonsten gilt der Konnektionstest als nicht bestanden und es wird angenommen, dass der arterielle Nadelanschluss 5 nicht mit der venösen Zugabestelle 7 konnektiert ist.

Zum erfindungsgemäßen Entfernen von Blut wird ein Prädilutionsventil oder -anschluss, vorgesehen zur Einleitung von Substituatflüssigkeit in die Blutleitung zwischen der Blutbehandlungseinrichtung 3000 und der Blutpumpe 4000 während einer Blutbehandlung, geöffnet. Die Substituatleitung ist mit dem Prädilutionsanschluss verbunden, so dass die zweite Fördereinrichtung 5000 Substituatlösung in einen zwischen der Blutpumpe 4000 und der Blutbehandlungseinrichtung 3000 gelegenen Abschnitt des extrakorporalen Blutkreislaufs 2000 einleiten kann.

Die Blutpumpe 4000 beginnt in der hier dargestellten Ausführungsform im selben Moment (gleichzeitig), in welchem mittels der zweiten Fördereinrichtung 5000 zu fördern begonnen wird, mit einer geringeren Förderrate als die zweite Fördereinrichtung 5000 rückwärts, d. h. in der zweiten Förderrichtung, zu fördern. Dabei wird der Substituatfluss aufgrund der unterschiedlichen Fördergeschwindigkeiten der beiden Pumpen - im Beispiel der Fig. 1 an einem Prädilutionsventil 33 - geteilt - ein Teilstrom der Substituatlösung bewegt sich zur Blutpumpe 4000 hin, ein anderer Teilstrom der Substituatlösung bewegt sich in Richtung auf die Blutbehandlungseinrichtung 3000 zu.

Die sich einstellenden venösen und/oder arteriellen Drücke werden während dieses Prozesses überwacht. Die eingestellten Pumpraten oder Förderraten der Fördereinrichtungen (Blutpumpe 4000 und zweite Fördereinrichtung 5000) können das Entfernen des Bluts entscheidend beeinflussen.

In bestimmten Ausführungsformen der vorliegenden Erfindung wird sichergestellt, dass die Förderrate der Blutpumpe 4000 nicht zu hoch gewählt wird. Damit kann vorteilhaft sichergestellt werden, dass das Blut beim Durchströmen der Einleitungsstelle - beispielsweise mit einem dünneren Schlauch und einem Rückschlagventil versehen - nicht geschädigt wird. Hierzu ist eine Begrenzung der maximalen Förderrate, beispielsweise aufgrund von Erfahrungswerten aus *in-vitro*- bzw. *in-vivo-Tests,* möglich oder vorgesehen.

In manchen Ausführungsformen der vorliegenden Erfindung ist eine Begrenzung und/oder Überwachung des Druckabfalls über der Zugabestelle und/oder der Zugabeleitung und dem Rückschlagventil 24 mit Hilfe des arteriellen Drucksensors 9 beim Reinfundieren möglich.

Beim Einstellen der "venösen" Reinfusionsrate, d. h. der Geschwindigkeit, mit der das extrakorporal vorliegende Blut über den venösen Leitungsabschnitt 3 des extrakorporalen Blutkreislaufs 2000 zum Patienten gefördert wird, wird in manchen erfindungsgemäßen Ausführungsformen sichergestellt, dass jener Teil des extrakorporalen Blutkreislaufs 2000, welcher sich von der Prädilutionsstelle oder dem Prädilutionsventil bis zur venösen Zugabestelle 7 erstreckt, nicht eher oder früher geleert ist, bevor auch der arterielle Leitungsabschnitt 1 des extrakorporalen Blutreislaufs 2000 geleert ist.

Hierdurch kann in bestimmten Ausführungsformen der vorliegenden Erfindung vorteilhaft eine weitere Vermischung von Blut (arteriell) und Substituatflüssigkeit (venös) und eine damit einhergehende unnötige Erhöhung des Reinfusionsvolumens mit den bekannten unangenehmen Folgen für den Patienten verhindert werden.

Da bei einem extrakorporalen Blutkreislauf (auch als Schlauchset bezeichnet) die Volumina der einzelnen Leitungsabschnitte bekannt sind, ist es in bestimmten Ausführungsformen der vorliegenden Erfindung vorgesehen, die maximal mögliche oder zulässige venöse Förderrate als die Förderate im venösen Leitungsabschnitt des extrakorporalen Blutkreislaufs 2000 zu berechnen. Die Berechnung kann wie oben beschrieben erfolgen.

Die venöse Förderrate wird in manchen Ausführungsformen der vorliegenden Erfindung eingestellt durch die Förderrate Q5000 der zweiten Fördereinrichtung 5000 ("Substituatpumpe") abzüglich der Förderrate Q4000 der Blutpumpe 4000. Hierzu wird auf die oben stehenden Formeln verwiesen.

Da bei einer solchen fixen Festlegung des Volumens der Blutbehandlungseinrichtung 3000 der arterielle Leitungsabschnitt 1 des extrakorporalen Blutkreislaufs 2000 früher oder schneller als der venöse Leitungsabschnitt 3 geleert werden kann, wird in bestimmten Ausführungsformen der vorliegenden Erfindung vorgeschlagen, die Blutpumpe 4000 zu stoppen, bevor Substituatflüssigkeit über die venöse Zugabestelle 7 hinweg gefördert wird.

Ein solches Stoppen der Förderung mittels der Blutpumpe 4000 ist in bestimmten Ausführungsformen bei Kenntnis des o. g. Volumens mittels entsprechenden Einstellens der Förderrate der Blutpumpe 4000 möglich. Ein - ggf. vorteilhaft noch genaueres - Abpassen des Moments, zu welchem die Blutpumpe 4000 gestoppt werden kann, ist auch unter Berücksichtigung der Signale des arteriellen Luftblasendetektors oder optischen Sensors 11 - soweit vorhanden - möglich.

In einigen erfindungsgemäßen Ausführungsformen wird mit dem Vorteil der Zeitersparnis ab dem Zeitpunkt des Anhaltens der Blutpumpe 4000 die Förderrate der zweiten Fördereinrichtung 5000 erhöht.

Ist das Volumen der jeweils eingesetzten Blutbehandlungseinrichtung 3000 bekannt, wird die Förderrate vorteilhaft bei jeder Reinfusion individuell auf die maximal mögliche Förderrate eingestellt. Dies verkürzt aufgrund der zügiger ablaufenden Entfernung von Blut aus dem extrakorporalen Blutkreislauf 2000 die Zeitdauer, die der Patient und das Bedienpersonal bis zum Abschluss dieser Maßnahme an der Blutbehandlungsvorrichtung verbringen müssen.

Der Typ der verwendeten Blutbehandlungseinrichtung 3000 kann vom Bedienpersonal eingestellt werden. Alternativ kann anhand bestimmter Parameter, welche beim Füllen der Blutbehandlungseinrichtung 3000 zu beobachten sind, der verwendete Typ automatisch ermittelt werden.

Ist das Volumen der Blutbehandlungseinrichtung 3000 bekannt, so wird in einigen erfindungsgemäßen Ausführungsformen die Förderrate der zweiten Fördereinrichtung 5000 derart eingestellt, dass an der Einleitstelle der venösen Zugabestelle 7 (oder einer vergleichbaren Stelle im Blutkreislauf) Substituat und Blut gleichen Verdünnungsgrades aus beiden Leitungsabschnitten 1, 3 zeitgleich aufeinandertrifft. Die Blutpumpe 4000 kann anschließend entweder angehalten werden; alternativ läuft sie weiter.

In Ausführungsformen, in denen die Blutpumpe 4000 weiterläuft, wird vorteilhaft der arterielle Leitungsabschnitt 1 des extrakorporalen Blutkreislaufs 2000 vergleichsweise besser durchspült; in Ausführungsformen, in denen die Blutpumpe 4000 gestoppt wird, wird vorteilhaft die Blutbehandlungseinrichtung 3000 vergleichsweise besser durchspült.

Die individuelle Einstellung der Förderraten geschieht in bestimmten Ausführungsformen gemäß der o. g. Formeln (II) und ggf. (III).

**Fig. 2** zeigt Druck- und Volumenverläufe (y-Achse, vertikal) über der Zeit (x-Achse, horizontal), welche beim Ausführen einer beispielhaften Ausführungsform des offenbarten Verfahrens mittels der in Fig. 1 gezeigten Anordnung auftreten oder gemessen werden können.

Die Skala links gibt ein Volumen in ml/min für Kurven Q4000 und Q5000 an, die Skalen rechts geben Drücke in mmHg an und gelten für Kurven P_art für den arteriellen Druckverlauf und P_ven für den venösen Druckverlauf, sowie für P_ven_sm, dem rechnerisch geglätteten Verlauf der Kurve P_ven.

Fig. 2 zeigt eine Kurve Q5000, welche das Fördervolumen der zweiten Fördereinrichtung 5000 über der Zeit t oder die Förderrate Q5000 angibt. Die Kurve Q5000 setzt sich aus mehreren Kurvenabschnitten Q5000-1 bis Q5000-5 zusammen.

Q5000-1 zeigt die Förderleistung der zweiten Fördereinrichtung 5000 während eines optionalen, sogenannten Freispülens des Prädilutionsventils 33. Die hierzu angelegte Förderleistung der zweiten Fördereinrichtung 5000 beträgt, ausgehend von 0 ml/min, beispielsweise 100 ml/min. Nach Abschluss dieses Freispülens kann die Förderleistung optional wieder auf 0 ml/min abfallen. Das dabei geförderte Volumen kann beispielsweise 10 ml betragen.

Q5000-2 gibt die Förderleistung während der erfindungsgemäßen Blutrückgabe oder -entleerung an. Q5000-2 hat rein exemplarisch einen treppenartig ansteigenden Verlauf, welcher allerdings weder treppenartig sein muss noch die hier gezeigten Schrittweiten und -höhen aufweisen muss. Q5000-2 zeigt die nach und nach ansteigende, von der Steuervorrichtung nach obiger Formel II sukzessive erhöhte Förderleistung Q5000_aktuell der zweiten Fördereinrichtung 5000.

Der Anstieg von Q5000, also der Abschnitt Q5000-2, beginnt zum Zeitpunkt t=0.

Der Verlaufsabschnitt Q5000-2 geht im vorliegenden Beispiel zu einem Zeitpunkt t=1 über in einen hier weiter ansteigenden Abschnitt Q5000-3. Die Dauer des Abschnitts Q5000-2 beträgt damit T = (t=1) - (t=0).

Die Förderleistung Q5000 der zweiten Fördereinrichtung 5000 steigt ab dem Zeitpunkt t=1 bis zum Erreichen einer vorbestimmten maximalen Förderleistung Q5000-4, hier exemplarisch 300 ml/min, wiederum treppenförmig an.

Die maximale Förderleistung Q5000-4 kann wiederum in Stufen oder treppenartig erreicht werden, wie in Fig. 2 beispielhaft gezeigt ist. Sie kann allerdings auch in einem senkrechten Anstieg (bezogen auf die Fig. 2) erreicht werden, oder auf andere Weise.

Ab dem Zeitpunkt t=1 wird eine Erhöhung von Q5000 ohne Blick auf den arteriellen Druck vorgenommen. Die Formeln II und III werden ab t=1 nicht mehr berücksichtigt. Die Erhöhung erfolgt ab t=1 ungeachtet des arteriellen Drucks.

Nach Erreichen und Halten der maximalen Förderleistung Q5000-4 fällt die Kurve Q5000 in ihrem Abschnitt Q5000-5 zum Zeitpunkt t=2 auf 0 ml/min ab. Das offenbarte Verfahren ist in t=2 beendet.

Fig. 2 zeigt weiter eine Kurve Q4000, welche das Fördervolumen der Blutpumpe 4000 über der Zeit oder die Förderrate Q4000 angibt. Die Kurve Q4000 setzt sich aus mehreren Kurvenabschnitten Q4000-1 bis Q4000-4 zusammen.

Q4000-1 zeigt die eingestellte Förderleistung und -dauer während eines optionalen Konnektionstests. Die angelegte Förderleistung beträgt, ausgehend von 0 ml/min, beispielsweise 25 ml/min. Nach Abschluss dieses Konnektionstests kann die Förderleistung optional wieder auf 0 ml/min abfallen.

Q4000-2 zeigt die Förderleistung während der erfindungsgemäßen Blutrückgabe oder -entleerung. Q4000-2 zeigt exemplarisch einen treppenartig ansteigenden Verlauf, welcher allerdings weder treppenartig sein muss noch die hier gezeigten Schrittweiten und -höhen aufweisen muss. Q4000-2 zeigt die nach und nach ansteigende Förderleistung der Blutpumpe 4000, welche in festem Verhältnis zur Förderleistung der zweiten Fördereinrichtung 5000 steht.

Der Anstieg von Q4000, also ihr Abschnitt Q4000-2, beginnt zum Zeitpunkt t=0.

Der Verlaufsabschnitt Q4000-2 geht zu einem Zeitpunkt t=1', welcher zwischen t=0 und t=1 liegt, über in ein Plateau, den Abschnitt Q4000-3. Das Plateau kann einer vorbestimmten maximalen Förderleistung von beispielsweise 100 ml/min entsprechen.

Die Förderleistung der Blutpumpe 4000 fällt zum Zeitpunkt t=1 auf 0 ml/min ab. Der Abfall ist mit Q4000-4 bezeichnet.

Fig. 2 zeigt ferner einen Druckverlauf P_art, seine Skalierung ist am rechten Rand der Fig. 2 dargestellt, und zwar in der linken der beiden benachbarten Skalen.

Der Druck P_art, beispielsweise mittels des arteriellen Drucksensors 9 der Fig. 1 gemessen, steigt mit Beginn der Förderung mittels der Blutpumpe 4000 ab dem Zeitpunkt t=0 an. Die lokalen Spitzenwerte P_art-1, P_art-2, usw. bezeichnen den jeweils höchsten Druck, der in bestimmten Drehwinkelbereichen bei Förderung mit der als Rollenpumpe ausgestalteten Blutpumpe 4000 gemessen wird. Dieser Spitzendruck tritt jeweils während des Eingriffs einer Rolle der Blutpumpe 4000 mit dem Pumpschlauchsegment auf. Der Spitzenwert ist veränderlich, wie auch Fig. 2 zeigt. Der jeweils zuletzt gemessene Spitzenwert P_art-1, P_art-2, usw. geht als P_art,scan in die Steuerung ein. So wird zunächst ein erster, unmittelbar nach Beginn des offenbarten Verfahrens auftretender Spitzenwert P_art-1 gemessen und im folgenden Beispiel als Grundlage der Berechnung als P_art,scan verwendet.

Die Blutpumpe 4000 erzeugt bauartbedingt pro Umdrehung zwei Druckpulse (könnten bei anderer Bauart mehr als zwei sein), welche am arteriellen Drucksensor 9 gemessen werden. Ein in der Steuereinrichtung hinterlegter Algorithmus ermittelt jeweils diese Spitzendrücke P_art-1, P_art-2, usw. anhand des Drehwinkels des Pumprotors und des aktuell am Drucksensor 9 anliegenden Drucks und stellt diesen für die weitere Verarbeitung als P_art,scan zur Verfügung, wie vorstehend beschrieben.

Die Blutrückgabe beginnt zu t=0, wenn die zweite Fördereinrichtung 5000 vorzugsweise gleichzeitig mit der Blutpumpe 4000 mit dem Fördern, optional mit der kleinstmöglichen Rate (die Begrenzung nach unten ist dabei bauartbedingt), in diesem Fall 25 ml/min, beginnt.

Bis zum Zeitpunkt t=1 fördern sowohl die zweite Fördereinrichtung 5000 als auch die Blutpumpe 4000.

Dabei wird die Blutpumpe 4000 stets derart angesteuert, um mit nur einem Bruchteil der Förderrate Q5000 der zweiten Fördereinrichtung 5000 zu fördern. Das hier exemplarisch ausgewählte Verhältnis beträgt Q4000 = Q5000*0,82. Dieser Wert hat sich als besonders vorteilhaft erwiesen, da bei ihm ein Verbringen von Gerinnseln vom Eingang der Blutbehandlungseinrichtung, hier des Dialysators, auf dessen Blutseite in den arteriellen Leitungsabschnitt 1 verhindert wird.

Dabei kann vorgesehen sein, dass ab einer Förderung Q5000 der zweiten Fördereinrichtung 5000 von beispielsweise mehr als 122 ml/min die Förderrate Q4000 der Blutpumpe 4000 auf beispielsweise 100 ml/min begrenzt ist. Dies kann vorteilhaft einer an der venösen Zugabestelle 7 auftretenden Hämolyse entgegenwirken oder diese begrenzen.

Nach jedem von der Blutpumpe 4000 erzeugten und am Drucksensor 9 gemessenen positiven Druckpuls P_art,scan wird die Förderrate Q5000 der zweiten Fördereinrichtung 5000 angehoben. Sie wird angehoben, bis eine obere, vom Anwender anhand der oberen Messbereichsgrenze des Drucksensors bestimmte Druckgrenze P_art,soll erreicht ist.

Der Druck am Drucksensor 9 soll dabei im vorliegenden Beispiel zuverlässig nicht über dessen obere Messbereichsgrenze P_art,max, hier 455 mmHg, steigen. P_art,max ist in diesem Beispiel jener Druckwert, ab welchem der Drucksensor 9 einen Alarm wegen zu hohen Drucks veranlasst. Daher wird für den einzuregelnden Druck am Drucksensor 9 das Regelungsziel P_art,soll auf 90% der Messbereichsgrenze festgelegt. P_art,soll beträgt damit Messbereichsgrenze*0,9, also rund 410 mmHg.

Im Beispiel der Fig. 2 beträgt der erste Spitzenwert für den Druck, nämlich P_art-1 etwa 260 mmHg. Gemessen am Zielwert P_art,soll von rund 410 mmHg (entspricht 455 mmHg * 0,9) beträgt die Regeldifferenz in diesem Beispiel nun rund 150 mmHg.

Die Förderrate Q5000 der zweiten Fördereinrichtung 5000 wird im Abschnitt Q5000-2 folgendermaßen erhöht:

$$Q5000\_künftig = Q5000\_aktuell + ((P\_art,soll)-(P\_art,scan))*0,12 *ml/min$$

Q5000_künftig ist dabei die neu einzustellende Förderrate. Q5000_aktuell ist die zuletzt eingestellte. Q5000_künftig könnte daher auch als Q5000(t=x+1) und Q5000_aktuell könnte als Q5000(t=x) bezeichnet werden.

Sollte sich der Druck am Drucksensor 9 über das Regelungsziel P_art,soll hinaus erhöhen, wird folgendermaßen gegengeregelt:

$$Q5000\_künftig = Q5000\_aktuell + ((P\_art,soll)-(P\_art,scan))*0,24 *ml/min$$

Ab dem Zeitpunkt t=1 der Fig. 2 fördert die Blutpumpe 4000 nicht mehr, es fördert nur noch die zweite Fördereinrichtung 5000.

Ab dem Zeitpunkt t=1 der Fig. 2 wird die Förderrate Q5000 der zweiten Fördereinrichtung 5000 hier rein beispielhaft alle 0,5s um 10ml/min erhöht, bis der vom Anwender am Gerät eingestellte Maximalfluss erreicht ist. Andere Werte sind selbstverständlich ebenfalls von der vorliegenden Erfindung umfasst.

Der Verlauf P_ven ist mittels des venösen Drucksensors gemessen, der Verlauf P_ven_sm entspricht einer Glättung des erstgenannten. Die Verläufe P_ven und P_ven_sm, zu welchen die rechteste aller Skalen gehört, bleiben vorliegend außer Betracht.

**Bezugszeichenliste**

| Bezugszeichen | Bezeichnung |
|---|---|
| 1000 | Funktionseinrichtung |
| 2000 | extrakorporaler Blutkreislauf |
| 3000 | Blutbehandlungseinrichtung |
| 4000 | Blutpumpe |
| 5000 | zweite Fördereinrichtung |
| 1 | arterieller Leitungsabschnitt |
| 3 | venöser Leitungsabschnitt |
| 5 | arterieller Nadelanschluss |
| 7 | venöse Zugabestelle |
| 9 | (arterieller) Drucksensor, Druckmesser |
| 11 | arterielle Klemme |
| 13 | arterielles Septum |
| 15 | venöser Luftblasendetektor/optischer Sensor |
| 17 | venöse Klemme |
| 19 | venöse Nadel |
| 21 | venöser Patientenkonnektor |

(fortgesetzt)

| Bezugszeichen | Bezeichnung |
|---|---|
| 23 | venöses Septum |
| 24 | Rückschlagventil |
| 25 | Heparinspritze |
| 27 | arterieller Luftblasendetektor/optischer Sensor |
| 29 | Single-Needle-Kammer |
| 29a | Blutkammer oder Blasenkammer |
| 31 | Gerinnselfänger |
| 33 | Prädilutionsventil |
| P art | arterieller Druck |
| P art-1, -2, -3 | Spitzenwerte P art,scan |
| P ven | venöser Druck |
| P ven sm | geglätteter Verlauf der Kurve P ven. |
| Q4000 | Förderrate der Blutpumpe |
| Q4000-1 bis Q4000-4 | Abschnitte der Kurve Q4000 |
| Q5000 | Förderrate der zweiten Fördereinrichtung |
| Q5000-1 bis Q5000-5 | Abschnitte der Kurve Q5000 |

**Patentansprüche**

1. Steuer- oder Regelungseinrichtung, konfiguriert zur Durchführung eines Verfahrens zum Entfernen von Blut aus einem extrakorporalen Blutkreislauf (2000) und/oder einer Funktionseinrichtung (1000), welche jeweils zum Zweck einer Blutbehandlung eines Patienten mit einer Blutbehandlungsvorrichtung verbunden ist bzw. sind, nach Beenden der Blutbehandlungssitzung, und im Zusammenwirken mit einer Blutbehandlungsvorrichtung, wobei die Blutbehandlungsvorrichtung aufweist oder verbunden ist mit:

   - wenigstens einen/m extrakorporalen Blutkreislauf (2000) mit einem Leitungsinneren, wobei der extrakorporale Blutkreislauf (2000) wenigstens einen arteriellen Leitungsabschnitt (1) und wenigstens einen venösen Leitungsabschnitt (3) aufweist;
   - wenigstens eine/r Blutpumpe (4000) zum Fördern von Blut innerhalb des Leitungsinneren;
   - wenigstens eine/r zweite(n) Fördereinrichtung (5000), insbesondere einer Substituatpumpe, zum Einbringen eines zweiten Fluids, insbesondere einer Substituatflüssigkeit, in das Leitungsinnere des extrakorporalen Blutkreislaufs (2000) und/oder zum Fördern eines Leitungsinhalts innerhalb des Leitungsinneren des extrakorporalen Blutkreislaufs (2000);

   wobei das Verfahren den Schritt umfasst:

   - Betreiben der Blutpumpe (4000) in einer zur während der Blutbehandlung üblichen ersten Förderrichtung dieser Pumpe entgegengesetzten zweiten Förderrichtung;

   wobei ein erster Abschnitt des arteriellen Leitungsabschnitts (1) mit einem zweiten Abschnitt des venösen Leitungsabschnitts (3) des extrakorporalen Blutkreislaufs (2000) in Fluidverbindung verbunden ist oder wird, und **dadurch gekennzeichnet, dass** die Förderrate (Q4000) der Blutpumpe (4000) eingestellt ist oder wird, um zu wenigstens einem Zeitpunkt oder während wenigstens eines Zeitraums (t=1-t=0) oder bis zum Erreichen einer vorbestimmten maximalen Förderrate (Q4000-3), auf welche die Förderrate (Q4000) der Blutpumpe (4000) begrenzt ist oder anwenderbedingt sein soll, zwischen 75 % und 90 %, vorzugweise zwischen 80 % und 84 %, besonders bevorzugt 82 % einer Förderrate (Q5000) der zweiten Fördereinrichtung (5000) zu betragen.

**2.** Steuer- oder Regelungseinrichtung nach Anspruch 1, wobei eine Förderrate (Q4000) der Blutpumpe (4000) auf einen, insbesondere durch den Anwender, vorbestimmten Wert begrenzt ist, vorzugsweise auf einen Wert zwischen 90 und 110 ml/min.

**3.** Steuer- oder Regelungseinrichtung nach einem der vorangegangenen Ansprüche, wobei die Förderraten (Q4000, Q5000) der Blutpumpe (4000) und der zweiten Fördereinrichtung (5000) während des Entfernens von Blut mittels Drucküberwachung und/oder Druckmessung und/oder Druckbegrenzung überwacht und/oder geregelt werden, insbesondere mittels mehrfachen Messens eines aktuellen Förderdrucks (P_art,scan) der Blutpumpe (4000), Ermitteln einer Regeldifferenz zwischen gemessenem Förderdruck der Blutpumpe (4000) und einem Zielwert (P_art,soll), und Anhebens der Förderrate (Q4000) der Blutpumpe (4000) entsprechend der Regeldifferenz.

**4.** Steuer- oder Regelungseinrichtung nach einem der vorangegangenen Ansprüche, wobei der aktuelle Förderdruck (P_aart,scan) bei Erreichen eines vorbestimmten Drehwinkels oder Drehwinkelbereichs eines Pumprotors der Blutpumpe (4000) abgegriffen, gemessen oder errechnet wird.

**5.** Steuer- oder Regelungseinrichtung nach einem der vorangegangenen Ansprüche, wobei die Förderrate (Q4000) der Blutpumpe (4000) und/oder die Förderrate (Q5000) der zweiten Fördereinrichtung (5000) nicht weiter erhöht werden, wenn der aktuelle Förderdruck (P_art,scan) den Zielwert (P_art,soll) erreicht oder übertroffen hat, wobei der Zielwert (P_art,soll) sich wie folgt ergibt:

$$P\_art,soll = a*P\_art,max$$

wobei gilt:

P_art,soll ist der Zielwert des mittels arteriellem Druckmesser (9) gemessenen Drucks;
a ist eine Konstante; sie liegt bevorzugt zwischen 0,85 und 0,95, besonders bevorzugt beträgt sie 0,9; und
P_art,max ist eine voreingestellte oder vorbestimmte Messbereichsgrenze oder ein zulässiger Maximalwert für den mittels des arteriellen Druckmessers (9) gemessenen Drucks; P_art,max ist vorzugsweise eine Alarmauslösegrenze.

**6.** Steuer- oder Regelungseinrichtung nach einem der vorangegangenen Ansprüche, mit dem Schritt:

- Erhöhen der Förderrate (Q5000) der zweiten Fördereinrichtung (5000) gemäß der Formel:

$$Q5000\_künftig = Q5000\_aktuell + ((P\_art,soll) - (P\_art,scan))*b;$$

wobei gilt:

Q5000_künftig ist eine jeweils künftige, festzusetzende Förderrate der zweiten Fördereinrichtung zu einem Zeitpunkt t_künftig;
Q5000_aktuell ist eine aktuelle Förderrate der zweiten Fördereinrichtung zu einem Zeitpunkt t_aktuell, welche durch Q5000_künftig abzulösen ist und dieser zeitlich jeweils vorausgeht, wobei t_aktuell vor t_künftig liegt; t_künftig könnte auch als t(x+1) und t_aktuell als t(x) bezeichnet werden;
P_art, soll ist ein voreingestellter Zielwert, welcher am arteriellen Druckmesser (9) anliegen soll;
P_art,scan ist ein Druckwert, welcher am arteriellen Druckmesser (9) während des Anliegens der Förderrate Q5000_aktuell gemessen wird; und
b ist eine Konstante; sie liegt bevorzugt zwischen 0,10 und 0,15, besonders bevorzugt beträgt sie 0,12.

**7.** Steuer- oder Regelungseinrichtung nach einem der vorangegangenen Ansprüche, mit dem Schritt:

- Senken der Förderrate (Q5000) der zweiten Fördereinrichtung (5000) wenn der Förderdruck (P_art,scan) den Zieldruck (P_art,soll) erreicht oder übersteigt, gemäß der Formel:

$$Q5000\_künftig = Q5000\_aktuell + ((P\_art,soll) - (P\_art,scan))*c;$$

wobei gilt:

Q5000_künftig ist eine jeweils künftige, festzusetzende Förderrate der zweiten Fördereinrichtung zu einem Zeitpunkt t_künftig;
Q5000_aktuell ist eine aktuelle Förderrate der zweiten Fördereinrichtung zu einem Zeitpunkt t_aktuell, welche durch Q5000_künftig abzulösen ist und dieser zeitlich jeweils vorausgeht, wobei t_aktuell vor t_künftig liegt; t_künftig könnte auch als t(x+1) und t_aktuell als t(x) bezeichnet werden;
P_art,soll ist ein voreingestellter Zielwert, welcher am arteriellen Druckmesser (9) anliegen soll;
P_aart,scan ist ein Druckwert, welcher am arteriellen Druckmesser (9) während des Anliegens der Förderrate Q5000_aktuell gemessen wird; und
c ist eine Konstante; sie liegt bevorzugt zwischen 0,20 und 0,30, besonders bevorzugt beträgt sie 0,24.

8. Steuer- oder Regelungseinrichtung nach einem der vorangegangenen Ansprüche mit dem Schritt:

   - Stoppen der Blutpumpe (4000), bevor Substituatflüssigkeit an der venösen Zugabestelle (7) in den venösen Leitungsabschnitt (3) des extrakorporalen Blutkreislaufs (2000) eingebracht wird, oder bevor Substituatflüssigkeit eine Stelle erreicht, an welcher sich Fluid aus dem venösen Leitungsabschnitt (3) und Fluid aus dem arteriellen Leitungsabschnitt (1) miteinander vereinen oder aufeinandertreffen; oder
   - Stoppen der zweiten Fördereinrichtung (5000) bevor Substituatflüssigkeit an der venösen Zugabestelle (7) in den venösen Leitungsabschnitt (3) des extrakorporalen Blutkreislaufs (2000) eingebracht wird, oder bevor Substituatflüssigkeit eine Stelle erreicht, an welcher sich Fluid aus dem venösen Leitungsabschnitt (3) und Fluid aus dem arteriellen Leitungsabschnitt (1) miteinander vereinen oder aufeinandertreffen.

9. Steuer- oder Regelungseinrichtung nach einem der vorangegangenen Ansprüche, ferner umfassend den Schritt:

   - Überprüfen der Konnektion des ersten Abschnitts des arteriellen Leitungsabschnitts (1), insbesondere des arteriellen Nadelanschlusses (5), des extrakorporalen Blutkreislaufs (2000) mit dem zweiten Abschnitt des venösen Leitungsabschnitts (3), insbesondere der venösen Zugabestelle (7), des extrakorporalen Blutkreislaufs (2000).

10. Steuer- oder Regelungseinrichtung nach Anspruch 9, wobei die Überprüfung umfasst:

    - Erzeugen eines Druckausgleichs;
    - Detektieren eines diastolischen Patientendrucks; und
    - Aufbauen eines Unterdrucks mittels der in erster Förderrichtung oder vorwärts fördernden Blutpumpe (4000).

11. Steuer- oder Regelungseinrichtung nach einem der vorangegangenen Ansprüche, wobei die venöse Zugabestelle (7) des venösen Leitungsabschnitts (3) des extrakorporalen Blutkreislaufs (2000) stromaufwärts einer Blutkammer (29a) und stromaufwärts eines Gerinnselfängers (31) in den venösen Leitungsabschnitt (3) mündet.

12. Blutbehandlungsvorrichtung, insbesondere als Hämodialysevorrichtung, Hämofiltrationsvorrichtung oder Hämodiafiltrationsvorrichtung ausgestaltet, mit einer Steuer- oder Regelungseinrichtung gemäß einem der Ansprüche 1 bis 11.

13. Digitales Speichermedium, insbesondere in Form einer Diskette, CD oder DVD oder EPROM, mit elektrisch auslesbaren Steuersignalen, konfiguriert, um derart mit einem programmierbaren Computersystem zusammenzuwirken, dass die maschinellen Schritte des in einem der Ansprüche 1 bis 11 beschriebenen Verfahrens mittels einer in einem der Ansprüche 1 bis 11 beschriebenen Blutbehandlungsvorrichtung veranlasst werden.

14. Computerprogramm-Produkt mit einem auf einem maschinenlesbaren Träger gespeicherten Programmcode zur Veranlassung der maschinellen Schritte des in einem der Ansprüche 1 bis 11 beschriebenen Verfahrens mittels einer in einem der Ansprüche 1 bis 11 beschriebenen Blutbehandlungsvorrichtung, wenn das Computerprogramm-Produkt auf einem Rechner abläuft.

15. Computerprogramm mit einem Programmcode zur Veranlassung der maschinellen Schritte des in einem der Ansprüche 1 bis 11 beschriebenen Verfahrens mittels einer in einem der Ansprüche 1 bis 11 beschriebenen Blutbehandlungsvorrichtung, wenn das Computerprogramm auf einem Computer abläuft.

**Claims**

1. A control or regulation device, configured to carry out a method for removing blood from an extracorporeal blood circuit (2000) and/or from a functional device (1000), which is or are respectively connected with a blood treatment apparatus for the purpose of treating a patient's blood, after the end of the blood treatment session, and in cooperation with a blood treatment apparatus,

   wherein the blood treatment apparatus comprises or is connected with:

   - at least one extracorporeal blood circuit (2000) with a line interior, the extracorporeal blood circuit (2000) comprising at least one arterial line section (1) and at least one venous line section (3);
   - at least one blood pump (4000) for conveying blood within the line interior;
   - at least one second conveying means (5000), in particular a substitute pump, for introducing a second fluid, in particular a substitute liquid into the line interior of the extracorporeal blood circuit (2000) and/or for conveying a line content within the line interior of the extracorporeal blood circuit (2000);

   wherein the method includes the step of:

   - operating the blood pump (4000) in a second conveying direction opposite to a usual first conveying direction of this pump during the blood treatment;

   wherein a first section of the arterial line section (1) is or will be connected in fluid communication with a second section of the venous line section (3) of the extracorporeal blood circuit (2000), and
   **characterized in that**
   the conveying rate (Q4000) of the blood pump (4000) is or will be adjusted to be between 75% and 90%, preferably between 80% and 84%, particularly preferably 82% of a conveying rate (Q5000) of the second conveying means (5000), at at least one point in time or during at least a period of time (t=1-t=0) or until a predetermined maximum conveying rate (Q4000-3) is reached, to which the conveying rate (Q4000) of the blood pump (4000) is limited or depend on the user.

2. The control or regulation device according to claim 1, wherein a conveying rate (Q4000) of the blood pump (4000) is limited, in particular by the user, to a predetermined value, preferably to a value between 90 and 110 ml/min.

3. The control or regulation device according to anyone of the preceding claims, wherein the conveying rates (Q4000, Q5000) of the blood pump (4000) and of the second conveying means (5000) are monitored and/or regulated during the removal of blood, by means of pressure monitoring and/or pressure measurement and/or pressure limitation, in particular by means of multiple measurements of a current conveying pressure (P art, scan) of the blood pump (4000), by determining a regulation difference between measured conveying pressure of the blood pump (4000) and a target value (P_art, soll), and by raising the conveying rate (Q4000) of the blood pump (4000) according to the regulation difference.

4. The control or regulation device according to anyone of the preceding claims, wherein the current conveying pressure (P_art,scan) is tapped, measured or calculated when a predetermined rotation angle or rotation angle range of a pump rotor of the blood pump (4000) is reached.

5. The control or regulation device according to anyone of the preceding claims, wherein the conveying rate (Q4000) of the blood pump (4000) and/or the conveying rate (Q5000) of the second conveying means (5000) is/are not further increased when the current conveying pressure (P_art, scan) has reached or exceeded the target value (P_art,soll), the target value (P_art,soll) being obtained as follows:

$$P\_art,soll = a*P\_art,max$$

wherein:

P_art,soll is the target value of the pressure measured by means of an arterial pressure gauge (9);
a is a constant; it is preferably between 0.85 and 0.95, particularly preferably it is 0.9; and
P_art,max is a preset or predetermined measuring range limit or a permissible maximum value for the pressure measured by means of the arterial pressure gauge (9); P_art,max is preferably an alarm trigger limit.

6. The control or regulation device according to anyone of the preceding claims, with the step of:

- increasing the conveying rate (Q5000) of the second conveying means (5000) according to the formula:

$$Q5000\_künftig = Q5000\_aktuell + ((P\text{-}art,soll) - (P\_art,scan))*b;$$

wherein:

Q5000_künftig is respectively a future conveying rate of the second conveying means to be determined at a time t_künftig;
Q5000_aktuell is a current conveying rate of the second conveying means at a time t_aktuell, which must be replaced by Q5000_künftig and which precedes this one every time, wherein t_aktuell lies before t_künftig; t_künftig could also be designated as t(x+1) and t_aktuell as t(x);
P_art,soll is a preset target value which should prevail at the arterial pressure gauge (9);
P_art,scan is a pressure value which is measured at the arterial pressure gauge (9) during the application of the conveying rate Q5000-aktuell; and
b is a constant; it lies preferably between 0.10 and 0.15, particularly preferably it is 0.12.

7. The control or regulation device according to anyone of the preceding claims, with the step of:

- lowering the conveying rate (Q5000) of the second conveying means (5000) when the conveying pressure (P_art,scan) reaches or exceeds the target pressure (P-art,soll), according to the formula:

$$Q5000\_künftig = Q5000\text{-}aktuell + ((P\text{-}art,soll) - (P\_art,scan))*c;$$

wherein:

Q5000_künftig is respectively a future conveying rate of the second conveying means to be determined at a time t_künftig;
Q5000_aktuell is a current conveying rate of the second conveying means at a time t_aktuell, which must be replaced by Q5000_künftig and which precedes this one every time, wherein t_aktuell lies before t_künftig; t_künftig could also be designated as t(x+1) and t_aktuell as t(x);
P_art,soll is a preset target value which should prevail at the arterial pressure gauge (9);
P_art,scan is a pressure value, which is measured at the arterial pressure gauge (9) during the application of the conveying rate Q5000-aktuell; and
c is a constant; it lies preferably between 0.20 and 0.30, particularly preferably it is 0.24.

8. The control or regulation device according to anyone of the preceding claims, with the step of:

- stopping the blood pump (4000) before the substitute liquid is introduced into the venous line section (3) of the extracorporeal blood circuit (2000) at the venous addition point (7), or before the substitute liquid reaches a position where fluid from the venous line section (3) and fluid from the arterial line section (1) join together or merge;
or

- stopping the second conveying means (5000) before the substitute liquid is introduced into the venous line section (3) of the extracorporeal blood circuit (2000) at the venous addition point (7), or before the substitute liquid reaches a point where fluid from the venous line section (3) and fluid from the arterial line section (1) join together or merge.

9. The control or regulation device according to anyone of the preceding claims, further comprising the step:

- checking the connection of the first section of the arterial line section (1), in particular of the arterial needle connection (5) of the extracorporeal blood circuit (2000) with the second section of the venous line section (3), in particular of the venous addition point (7), of the extracorporeal blood circuit (2000).

10. The control or regulation device according to claim 9, wherein the check includes:

- creating a pressure balance;
- detecting a patient diastolic pressure; and
- building up an negative pressure by means of the blood pump (4000) conveying in the first conveying direction or forward.

11. The control or regulation device according to anyone of the preceding claims, wherein the venous addition point (7) of the venous line section (3) of the extracorporeal blood circuit (2000) leads into the venous line section (3) upstream of a blood chamber (29a) and upstream of a clot trap (31).

12. A blood treatment apparatus, designed in particular as a hemodialysis apparatus, hemofiltration apparatus or hemodiafiltration apparatus, having a control or regulation device according to anyone of claims 1 to 11.

13. A digital storage medium, in particular in the form of a floppy disk, CD or DVD or EPROM, with electrical readable control signals, configured to interact with a programmable computer system such that the mechanical steps of the method described in anyone of claims 1 to 11 are initiated by means of a blood treatment apparatus described in anyone of claims 1 to 11.

14. A computer program product having a program code stored on a machine-readable carrier for initiating the mechanical steps of the method described in anyone of claims 1 to 11 by means of a blood treatment apparatus described in anyone of claims 1 to 11, when the computer program product runs on a computer.

15. A computer program having a program code for initiating the mechanical steps of the method described in anyone of claims 1 to 11 by means of a blood treatment apparatus described in anyone of claims 1 to 11, when the computer program runs on a computer.

**Revendications**

1. Un dispositif de commande ou de régulation, configuré pour exécuter un procédé d'élimination du sang d'un circuit sanguin extracorporel (2000) et/ou d'un dispositif fonctionnel (1000), qui est, ou sont, respectivement relié (s) à un appareil de traitement du sang dans le but de traiter le sang d'un patient, après la fin de la séance de traitement du sang, et en coopération avec un appareil de traitement du sang,
où l'appareil de traitement du sang comprend ou est relié à:

- au moins un circuit sanguin extracorporel (2000) avec un intérieur de conduit, le circuit sanguin extracorporel (2000) comprenant au moins une section de conduit artériel (1) et au moins une section de conduit veineux (3);
- au moins une pompe à sang (4000) pour acheminer le sang dans le conduit intérieur;
- au moins un second dispositif d'acheminement (5000), notamment une pompe à substitut, pour introduire un second fluide, notamment un liquide de substitut, dans l'intérieur de conduit du circuit sanguin extracorporel (2000) et/ou pour acheminer un contenu de conduit dans l'intérieur de conduit du circuit sanguin extracorporel (2000);

où le procédé inclut l'étape consistant à:

- faire fonctionner la pompe à sang (4000) dans une seconde direction d'acheminement opposée à une première direction d'acheminement habituelle de cette pompe pendant le traitement du sang;

où une première partie de la section de conduit artériel (1) est ou sera reliée en communication fluidique avec une seconde partie de la section de conduit veineux (3) du circuit sanguin extracorporel (2000), et
**caractérisé en ce que**
le débit d'acheminement (Q4000) de la pompe à sang (4000) est ou sera ajusté pour être compris entre 75% et 90%, de préférence entre 80% et 84%, plus préférablement à 82% d'un débit d'acheminement (Q5000) du second dispositif d'acheminement (5000), à au moins un moment donné ou pendant au moins une période de temps (t=1-t=0) ou jusqu'à ce qu'un débit d'acheminement maximal prédéterminé (Q4000-3) soit atteint, auquel le débit d'acheminement (Q4000) de la pompe à sang (4000) est limité ou dépend de l'utilisateur.

2. Le dispositif de commande ou de régulation selon la revendication 1, où le débit d'acheminement (Q4000) de la pompe à sang (4000) est limité, notamment par l'utilisateur, à une valeur prédéterminée,
de préférence à une valeur comprise entre 90 et 110 ml/min.

3. Le dispositif de commande ou de régulation selon l'une quelconque des revendications précédentes, où les débits d'acheminement (Q4000, Q5000) de la pompe à sang (4000) et du second dispositif d'acheminement (5000) sont surveillés et/ou régulés pendant l'élimination du sang au moyen d'une surveillance de pression et/ou d'une mesure de pression et/ou d'une limitation de pression, notamment au moyen de multiples mesures d'une pression actuelle d'acheminement (P_art,scan) de la pompe à sang (4000), en déterminant une différence de régulation entre la pression d'acheminement mesurée de la pompe à sang (4000) et une valeur cible (P_art,soll), et en augmentant le débit d'acheminement (Q4000) de la pompe à sang (4000) en fonction de la différence de régulation.

4. Le dispositif de commande ou de régulation selon l'une quelconque des revendications précédentes, où la pression d'acheminement actuelle (P_art, scan) est relevée, mesurée ou calculée lorsqu'un angle de rotation ou une plage d'angles de rotation prédéterminé(e) d'un rotor de pompe de la pompe à sang (4000) est atteint(e).

5. Le dispositif de commande ou de régulation selon l'une quelconque des revendications précédentes, où le débit d'acheminement (Q4000) de la pompe à sang (4000) et/ou le débit d'acheminement (Q5000) du second dispositif d'acheminement (5000) n'est/ne sont pas augmenté(s) davantage quand la pression d'acheminement actuelle (P_art,scan) a atteint ou dépassé la valeur cible (P_art,soll), la valeur cible (P_art,soll) étant obtenue comme suit:

$$P\_art,soll = a*P\_art,max$$

où:

P_art,soll est la valeur cible de la pression mesurée à l'aide d'un manomètre artériel (9);
a est une constante; elle est de préférence comprise entre 0,85 et 0,95, et plus particulièrement égale à 0,9; et
P_art,max est une limite de plage de mesure préréglée ou prédéterminée ou une valeur maximale admissible pour la pression mesurée à l'aide du manomètre artériel (9); P_art,max est de préférence une limite de déclenchement d'alarme.

6. Le dispositif de commande ou de régulation selon l'une quelconque des revendications précédentes, avec l'étape consistant à:

- augmenter le taux d'acheminement (Q5000) du second dispositif d'acheminement (5000) selon la formule suivante:

$$Q5000\_künftig = Q5000\_aktuell + ((P\text{-}art,soll)-(P\_art,scan))*b;$$

où:

Q5000_künftig est respectivement un taux d'acheminement futur, à déterminer, du second dispositif d'acheminement à un instant t_künftig;
Q5000_aktuell est un débit d'acheminement actuel du second dispositif d'acheminement à un instant t_aktuell, qui doit être remplacé par Q5000_künftig et qui précède celui-ci à chaque fois dans le temps, où t_aktuell se situe avant t_künftig; t_künftig pourrait également être désigné comme t(x+1) et t_aktuell comme t(x);

P_art,soll est une valeur cible préréglée qui doit prévaloir au manomètre artériel (9);

P_art,scan est une valeur de pression, qui est mesurée au manomètre artériel (9) pendant l'application du débit d'acheminement Q5000-aktuell; et

b est une constante; elle est comprise de préférence entre 0,10 et 0,15, plus particulièrement égale à 0,12.

7.  Le dispositif de commande ou de régulation selon l'une quelconque des revendications précédentes, avec l'étape consistant à:

- abaisser le débit d'acheminement (Q5000) du second dispositif d'acheminement (5000) lorsque la pression d'acheminement (P_art,scan) atteint ou dépasse la pression cible (P_art,soll), selon la formule suivante:

```
Q5000_künftig = Q5000-aktuell + ((P-art,soll)-
(P_art,scan))*c;
```

où:

Q5000_künftig est respectivement un taux d'acheminement futur, à déterminer, du second dispositif d'acheminement à un instant t_künftig;

Q5000_aktuell est un débit d'acheminement actuel du second dispositif d'acheminement à un instant t_aktuell, qui doit être remplacé par Q5000_künftig et qui précède celui-ci à chaque fois dans le temps, où t_aktuell se situe avant t_künftig; t_künftig pourrait également être désigné comme t(x+1) et t_aktuell comme t(x);

P_art,soll est une valeur cible préréglée qui doit prévaloir au manomètre artériel (9);

P_art,scan est une valeur de pression, qui est mesurée au manomètre artériel (9) pendant l'application du débit d'acheminement Q5000-aktuell; et

c est une constante; elle est comprise de préférence entre 0,20 et 0,30, plus particulièrement égale à 0,24.

8.  Le dispositif de commande ou de régulation selon l'une quelconque des revendications précédentes, avec l'étape consistant à:

- arrêter la pompe à sang (4000) avant que le liquide de substitut ne soit introduit dans la section de conduit veineux (3) du circuit sanguin extracorporel (2000) au niveau du point d'addition veineux (7), ou avant que le liquide de substitut n'atteigne une position où le fluide de la section de conduit veineux (3) et le fluide de la section de conduit artériel (1) s'unissent ou se rencontrent;
ou
- arrêter le second dispositif d'acheminement (5000) avant que le liquide de substitut ne soit introduit dans la section de conduit veineux (3) du circuit sanguin extracorporel (2000) au niveau du point d'addition veineux (7) , ou avant que le liquide de substitut n' atteigne une position où le fluide de la section de conduit veineux (3) et le fluide de la section de conduit artériel (1) s'unissent ou se rencontrent.

9.  Le dispositif de commande ou de régulation selon l'une quelconque des revendications précédentes, comprenant en outre l'étape consistant à:

- vérifier la liaison de la première partie de la section de conduit artériel (1), notamment du raccordement de l'aiguille artérielle (5), du circuit sanguin extracorporel (2000) avec la seconde partie de la section de conduit veineux (3), notamment du point d'addition veineux (7), du circuit sanguin extracorporel (2000).

10. Le dispositif de commande ou de régulation selon la revendication 9, où la vérification comprend:

- la création d'un équilibrage de pression;
- la détection de la pression diastolique du patient; et
- l'établissement d'une sous-pression au moyen de la pompe à sang (4000) acheminant dans la première direction d'acheminement ou vers l'avant.

**11.** Le dispositif de commande ou de régulation selon l'une quelconque des revendications précédentes, où le point d'addition (7) de la section de conduit veineux (3) du circuit sanguin extracorporel (2000) débouche dans la section de conduit veineux (3) en amont d'une chambre à sang (29a) et en amont d'un piège à caillot (31).

**12.** Un appareil de traitement du sang, conçu notamment comme un appareil d'hémodialyse, un appareil d'hémofiltration ou un appareil d'hémodiafiltration, doté un dispositif de commande ou de régulation selon l'une des revendications 1 à 11.

**13.** Un support de stockage numérique, notamment sous la forme d'une disquette, d'un CD, d'un DVD ou d'un EPROM, avec des signaux de commande électriques lisibles, configuré pour interagir avec un système informatique programmable, de telle sorte que les étapes mécaniques du procédé décrit dans l'une quelconque des revendications 1 à 11 soient initiées au moyen d'un appareil de traitement du sang décrit dans l'une quelconque des revendications 1 à 11.

**14.** Un produit de programme informatique ayant un code de programme stocké sur un support lisible par machine pour initier les étapes mécaniques du procédé décrit dans l'une quelconque des revendications 1 à 11 au moyen d'un appareil de traitement du sang décrit dans l'une quelconque des revendications 1 à 11, lorsque le produit de programme informatique s'exécute sur un ordinateur.

**15.** Un programme informatique ayant un code de programme pour initier les étapes mécaniques du procédé décrit dans l'une quelconque des revendications 1 à 11 au moyen d'un appareil de traitement du sang décrit dans l'une quelconque des revendications 1 à 11, lorsque le produit de programme s'exécute sur un ordinateur.

**Fig. 1**

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2010184029 A
- DE 102009027195 A1
- WO 2013017240 A2
- EP 2535065 A1
- EP 2535067 A1
- DE 102009018664 A1
- DE 102009024468 A1